# EUROPEAN PATENT APPLICATION

(11) **EP 3 838 269 A1**
(43) Date of publication of application: **23.06.2021**
(21) Application number: 19216548.8
(22) Date of filing: 16.12.2019
(51) Int. Cl.: A61K 31/00, A61K 31/65, A61K 31/7048, A61K 31/7052, A61K 45/06, A61P 1/00, A61P 1/14, A61K 31/05, A61K 31/343, A61K 31/37, A61K 31/7056, A61K 31/196, A61K 31/197, A61K 31/4152

(54) **COMPOUNDS AND PHARMACEUTICAL COMPOSITIONS FOR PREVENTION AND/OR TREATMENT OF DYSBIOSIS AND ANTIBACTERIAL ANTIDOTES FOR MICROBIOME-PROTECTION**

(71) Applicant: European Molecular Biology Laboratory, 69117 Heidelberg (DE)
(72) Inventor: Typas, Athanasios, 69120 Heidelberg (DE); Maier, Lisa, 72076 Tübingen (DE); Goemans, Camilie, 69117 Heidelberg (DE); Gontao Brochado, Ana Rita, 97246 Eibelstadt (DE); Cacace, Elisabetta, 53100 Siena (IT)
(74) Representative: Krauss, Jan

(57) **Abstract**

The present invention relates to the field of therapeutics and, more in particular, to pharmaceutical compositions for the prevention and/or treatment of bacterial infections and antibacterial-induced dysfunctions. The invention is based on the identification of compounds that prevent antibacterial effects of antibiotics on microbiome-bacterial species, while retaining antibacterial effects of antibiotics on pathogenic bacteria. The invention also pertains to pharmaceutical compositions comprising antibiotics and antibiotic antidotes for the protection of commensal microbiome-bacteria. Thus, this invention relates to compounds and pharmaceutical combinations useful to prevent antibiotic-induced adverse effects on the gut microbiome. Furthermore provided are methods for preventing an adverse effect on a gut microbiome using the compounds and/or pharmaceutical compositions of this invention.

## Description

### FIELD OF THE INVENTION

The present invention relates to the field of therapeutics and, more in particular, to pharmaceutical compositions for the prevention and/or treatment of bacterial infections and antibacterial-induced dysfunctions. The invention is based on the identification of compounds that prevent antibacterial effects of antibiotics on bacterial species present in the human microbiome, while retaining antibacterial effects of antibiotics on pathogenic bacteria. The invention also pertains to pharmaceutical compositions comprising antibiotics and antibiotic antidotes for the protection of commensal gut bacteria. Thus, this invention relates to compounds and pharmaceutical combinations useful to prevent antibiotic-induced adverse effects on the gut microbiome. Furthermore, provided are methods for preventing an adverse effect on a gut microbiome using the compounds and/or pharmaceutical compositions of this invention.

### DESCRIPTION

Medication is emerging as major contributor for changes in the composition of the human gut microbiota, and antibiotics govern the risk of harming the healthy intestinal flora. Severe and long-lasting changes in the composition of the human gut microbiota are associated, and are in some cases even causatively linked, to dysbiosis and a wide range of diseases. Although several non-antibiotic drugs may also have a previously unappreciated impact on the gut microbiome composition, antibiotics, developed to have broad spectra and thereby target very diverse pathogens, are known to take a heavy toll on our gut flora, causing a variety of gastrointestinal side-effects, including *Clostridioides (former Clostridium) difficile* infections.

Recently, more attention has been given to this collateral damage of antibiotics on the gut microbiota and thereby, on the host's wellbeing with *in vivo* studies highlighting links between the long-term microbiota compositional changes and host dysbiosis, including the development of allergic, metabolic, immunological and inflammatory diseases. While uncovering the direct effects of different antibiotics on the gut flora is critical to improve general health, technical difficulties hamper routine testing of antibiotic susceptibility in anaerobes.

Currently available data on bacterial susceptibility to antibiotics offers little to no resolution in the diversity of the human gut microbiota, and information is missing even for the most prevalent and abundant species, or species associated with dysbiosis and disease. Current methods for retaining healthy microbiota upon antibiotic therapies suffer from many limitations and are difficult to investigate and improve.

WO2014197562 (A1) provides methods for microbiota restoration therapies based on collecting human fecal samples and performing fecal transplants.

WO0207741 (A1) provides methods for a probiotic recolonisation therapy comprising recolonizing a dysbiotic enteric microflora in a subject in need thereof by administering a pharmaceutical composition comprising viable non-pathogenic or attenuated pathogenic Clostridia, viable non-pathogenic or attenuated pathogenic Bacteroides, and viable non-pathogenic or attenuated pathogenic Escherichia coli. Said viable non-pathogenic or attenuated pathogenic bacteria are capable of recolonizing a dysbiotic enteric microflora in a subject.

Langelier *et al.,* 2018 (Langelier et al. A proactive charcoal approach shields the gut microbiome from systemic antibiotics. Science Translational Medicine; 28 Feb 2018) discloses a method for gut microbiota protection against antibiotics using co-administration of activated charcoal.

Pitout *et al.,* 2009 (Pitout et al. IPSAT P1A, a class A beta-lactamase therapy for the prevention of penicillin-induced disruption to the intestinal microflora. Curr. Opin. Investig. Drugs. 2009 Aug; 10(8):838-44) discloses a method for the protection of the gut microbiota against antibiotics using co-administration of beta-lactamase.

However, previous methods for retaining healthy microbiota upon antibiotic therapies are not very efficient. For example, the use of fecal transplants has many limitations, such as the invasive nature and no guarantee for colonization upon fecal transplantion. Moreover, many patients are reluctant to receiving fecal transplants, and methods based on the use of fecal samples suffer from reservation against collection. The use of proactive activated charcoal or β-lactamase for protection of the gut microbiota against antibiotics suffers from inconveniences, such as high costs due to handling and storage. Moreover, while activated charcoal and β-lactamases show activities to some specific antibiotics, activated charcoal and β-lactamases are not very active against multiple other antibiotics. Thus, the use of activated charcoal and β-lactamases is very limited.

Due to the continuing need for methods to protect the gut microbiota from the effect of antibiotics, it is an objection of the present invention to provide compounds and/or pharmaceutical compositions that selectively antagonize the effect of antibiotics on gut microbes. It is a further object of this invention to develop antibacterial therapies that can prevent an adverse impact of antibiotic compounds selectively on healthy microorganisms residing in the patients' body, such as the gut microbiota, while retaining antibiotic activity of antibiotic compounds against pathogens. It is an additional object of this invention to develop compounds and pharmaceutical compositions useful in selectively protecting the gut microbiota from the adverse effects of antibiotic therapies, and methods for using the same. Yet another object of this invention to develop therapies for treating dysbiosis.

### BRIEF DESCRIPTION OF THE INVENTION

Generally, and by way of brief description, the main aspects of the present invention can be described as follows:
In **a first aspect**, the invention pertains to a compound that can antagonize the effect of antibiotics on gut microbes, while retaining activity against pathogenic bacteria.
In **a second aspect**, the invention pertains to a compound for use in treating and/or preventing dysbiosis.
In **a third aspect**, the invention pertains to pharmaceutical compositions comprising at least one antibiotic and at least one compound that can antagonize the effect of the at least one antibiotic on gut microbes, while retaining activity against pathogenic bacteria.
In **a fourth aspect**, the invention pertains to the use of the compounds or pharmaceutical compositions in the prevention and/or treatment of a disease in a subject, and/or the use in the prevention of a modification of the microbiome of said subject.
In **a fifth aspect**, the invention pertains to a method for preventing an adverse effect on a gut microbiome using the compounds or pharmaceutical compositions of this invention.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect thereof, the object of the present invention is solved by providing a compound, or a pharmaceutically acceptable salt thereof, wherein said compound, or the pharmaceutically acceptable salt thereof, is for use in treating and/or preventing dysbiosis, and/or for use in antagonizing an antibacterial effect of at least one second compound in at least one first bacterium, wherein said compound, or the pharmaceutically acceptable salt thereof, is capable of antagonizing an antibacterial effect of at least one second compound in at least one first bacterium, and wherein said compound, or the pharmaceutically acceptable salt thereof, is not capable of antagonizing an antibacterial effect of said at least one second compound in at least one second bacterium.

According to this invention, said compound to be administered to a subject in addition to said at least one second compound can be any compound. Preferably, said compound to be administered to a subject in addition to said at least one second compound is selected from an anticoagulant drug, a non-steroidal anti-inflammatory drug, an uricosuric, a nonsteroidal estrogen compound, an antagonist of the cholecystokinin CCKA receptor, a salicylic acid derivative, a cyclooxygenase-2 inhibitor, a cholecystographic agent, an angiotensin II receptor blocker, a thyroid hormone analogue, an antibacterial agent, an antifungal agent, or a pharmaceutically acceptable salt thereof.

In a preferred embodiment, said compound, or the pharmaceutically acceptable salt thereof, to be administered to a subject in addition to said at least one second compound, is selected from an anticoagulant drug, such as Dicumarol, a non-steroidal anti-inflammatory drug, such as Famprofazone, Tolfenamic acid, Celecoxib, Clonixin, or Meclofenamic acid, an Uricosuric, such as Benzbromarone, a nonsteroidal estrogen compound, such as Diethylstilbestrol or Hexestrol, an antagonist of the Cholecystokinin CCKA receptor, such as Lorglumide, a salicylic acid derivative, such as Diflunisal, a cholecystographic agent, such as Iopanoic acid, an angiotensin II receptor blocker, such as Losartan, a thyroid hormone analogue, such as Tiratricol, an antibacterial, antifungal and/or antibacterial agent, such as Triclosan or Diclazuril, a reverse transcriptase inhibitor, such as Efavirenz, a Ca-channel inhibitor, a human-targeted drug, and a food additive, or a pharmaceutically acceptable salt thereof.

In yet another preferred embodiment said compound or the pharmaceutically acceptable salt thereof to be administered to a subject in addition to said at least one second compound is a non-steroidal anti-inflammatory drug, preferably wherein said non-steroidal anti-inflammatory drug is Famprofazone, Clonixin, or a cyclooxygenase-2 inhibitor, more preferably wherein said cyclooxygenase-2 inhibitor is Tolfenamic acid, Celecoxib, or Meclofenamic acid.

In an even more preferable embodiment of this invention, said compound to be administered to a subject in addition to said at least one second compound is selected from Dicumarol, Tolfenamic acid, Famprofazone, Celecoxib, Benzbromarone, Diethylstilbestrol, Hexestrol, Lorglumide, Diflunisal, Iopanoic acid, Losartan, Tiratricol, Triclosan, Clonixin, Meclofenamic acid, Diclazuril, and Efavirenz, or a pharmaceutically acceptable salt thereof.

In a second aspect, the object of the present invention is solved by providing a compound, or a pharmaceutically acceptable salt thereof, wherein said compound, or the pharmaceutically acceptable salt thereof, is capable of antagonizing an antibacterial effect of at least one second compound in at least one first bacterium, and wherein said compound, or the pharmaceutically acceptable salt thereof, is not capable of antagonizing an antibacterial effect of at least one second compound in at least one second bacterium.

As used herein, "antagonism" or "antagonistic effect" refers to the effect that occurs when at least two compounds interact, and result in an overall effect that is lower than the sum of individual effects of either of them used alone. An "antagonistic" combination, as used herein, can prevent the antibacterial effect of one of the compounds used alone. Stated another way, antagonistic effect means that if at least one of the compounds is having an antibacterial effect against a bacterium or a bacterial species, this antibacterial effect can be counteracted by the at least one other compound. The at least one other compound can thus be seen as an "antidote" to said first compound. Particularly preferred is that the antagonistic effect occurs in a commensal bacterial species only, e.g. a human gut bacterial species, but does not occur in a pathogenic bacterial species. The combination of the at least two compounds is referred to as having an "antagonistic effect" if the antibacterial effect of at least one of the two compounds is masked when used in combination with the other compound in at least one bacterial species. "Antagonizing", in the context of the present invention, refers to having an "antagonistic effect". Stated another way, antagonistic effect means that if at least one of the compounds is having an antibacterial effect against a bacterium or a bacterial species, this antibacterial effect is counteracted, i.e. "antagonized", by the at least one other compound.

The term "synergy" or "synergistic effect" shall refer to the effect that occurs when at least two compounds interact, and result in an overall effect that is greater than the sum of individual effects of either of said compounds used alone. This combination thus greatly improves the antibacterial effect of one of the compounds used alone. Stated another way, synergistic effect means that the total antibacterial effect against a bacterium or a bacterial species of the combination of the two components is greater than the sum of the antibacterial effect of each component when measured separately.

The term "antibacterial" or "antibacterial effect" as used herein refers to the general term for bacteriostatic and bactericidal effects. The term "bacteriostatic" or "bacteriostatic action" refers to the inhibition of the growth and reproduction of microorganisms. As used herein, the term "bactericidal" or "bactericidal effect" shall refer to the role of killing microbial trophozoites and propagules. The term "antibacterial effective amount" as used herein refers to an antibacterial amount capable of achieving a desired antibacterial effect. Generally, depending on the antimicrobial requirements, the antimicrobial effects that need to be achieved may vary. The skilled artisan is aware of ways to determine the antibacterial effective amount of an antibacterial compound needed for achieving the desired antibacterial effect.

Importantly, many bacterial species residing in a person's or a patient's body are commensal or probiotic bacterial species. However, compounds having an antibiotic effect are often not only effective against pathogenic bacteria but also effective against commensal or probiotic bacterial species, thereby damaging the human or animal body, e.g. by harming the healthy gut microbiome. It is thus important to prevent such damaging effects on commensal and/or probiotic bacteria, while simultaneously enabling an antibiotic effect on pathogenic bacteria, which are causative for an infection and/or a disease.

The term "microbiota" refers, collectively, to the entirety of microbes found in association with a higher organism, such as a human. Organisms belonging to a human's microbiota may generally be categorized as bacteria, archaea, yeasts, and single-celled eukaryotes, as well as viruses and various parasites.

The term "microbiome" refers, collectively, to the entirety of microbes, their genetic elements (genomes), and environmental interactions, found in association with a higher organism, such as a human. Preferably, the term "microbiome" in the context of this invention shall refer to the commensal microbiome of a subject.

The microbiome comprises many commensal and/or probiotic bacterial strains. The term "commensal" refers to organisms that are normally harmless to a host, and can also establish mutualistic relations with the host. The human body contains about 100 trillion commensal organisms, which have been suggested to outnumber human cells by a factor of 10.

The term "probiotic" as used herein means living microorganisms, which when administered in adequate amounts, confer a health benefit on the host. Probiotics may be available in foods and dietary supplements (for example, but not limited to capsules, tablets, and powders). Examples of food containing probiotics are yogurt, fermented and unfermented milk, miso, tempeh, some juices and soy beverages. Some bacterial strains of the microbiome are known to have a probiotic function, such as *Lactobacillus, Bifidobacterium, Enterococcus, Streptococcus, Pediococcus, Leuconostoc, Bacillus, Escherichia, and Lactococcus.*

The term "dysbiosis" (also called dysbacteriosis) shall refer to any kind of imbalance of the microbiome. For example, species that are normally underrepresented in the microbiome of a healthy human being become overrepresented during the condition of dysbiosis, whereas normally dominated species of a healthy human being become underrepresented during the condition of dysbiosis. Most often, dysbiosis is a condition in the gastrointestinal tract, particularly during small intestinal bacterial overgrowth (SIBO) or small intestinal fungal overgrowth (SIFO). Dysbiosis has been reported to be associated with illnesses, such as inflammatory bowel disease, bacterial vaginosis, and colitis.

The inventors identified an antagonistic antibacterial effect of at least one compound on at least one first bacterium, wherein said first bacterium is a commensal bacterium and/or a probiotic bacterium, while said at least one compound is not having an antagonistic antibacterial effect on at least one second bacterium, wherein said second bacterium is a pathogenic bacterium. Thus, the at least one first compound is not preventing an antibacterial effect of the at least one second compound on a pathogenic bacterium, while the at least one first compound is preventing and/or relieving collateral damage to at least one commensal bacterium and/or a probiotic bacterium. The use of said at least one first compound in combination with said at least one second compound is thus advantageous over using one of the compounds alone, since the combination of compounds is preventing the damage of said compound having an antibacterial effect on a commensal bacterium and/or a probiotic bacterium.

In a preferred embodiment of this invention, said compound and said at least one second compound are administered to a subject simultaneously, separately, or sequentially. In particular, said at least one first compound and said at least one second compound are administered to a subject simultaneously, separately, or sequentially.

A further preferred embodiment relates to the compound of this invention, wherein said compound is administered to the subject by any suitable means including oral, intravenous, arterial infusion, intraperitoneal, parenteral, enteral, topical, intramuscular, subcutaneous, surgical, topical, cutaneous, subcutaneous, or any other clinically approved way of administration.

Said compound to be administered to a subject in addition to said at least one second compound can be any compound. Preferably, said compound to be administered to a subject in addition to said at least one second compound is selected from an anticoagulant drug, a non-steroidal anti-inflammatory drug, an uricosuric, a nonsteroidal estrogen compound, an antagonist of the cholecystokinin CCKA receptor, a salicylic acid derivative, a cyclooxygenase-2 inhibitor, a cholecystographic agent, an angiotensin II receptor blocker, a thyroid hormone analogue, an antibacterial agent, an antifungal agent, or a pharmaceutically acceptable salt thereof.

In a preferred embodiment, said compound, or the pharmaceutically acceptable salt thereof, to be administered to a subject in addition to said at least one second compound, is selected from an anticoagulant drug, such as Dicumarol, a non-steroidal anti-inflammatory drug, such as Famprofazone, Tolfenamic acid, Celecoxib, Clonixin, or Meclofenamic acid, an Uricosuric, such as Benzbromarone, a nonsteroidal estrogen compound, such as Diethylstilbestrol or Hexestrol, an antagonist of the Cholecystokinin CCKA receptor, such as Lorglumide, a salicylic acid derivative, such as Diflunisal, a cholecystographic agent, such as Iopanoic acid, an angiotensin II receptor blocker, such as Losartan, a thyroid hormone analogue, such as Tiratricol, an antibacterial, antifungal and/or antibacterial agent, such as Triclosan or Diclazuril, a reverse transcriptase inhibitor, such as Efavirenz, a Ca-channel inhibitor, a human-targeted drug, a food additive, or a pharmaceutically acceptable salt thereof.

In yet another preferred embodiment said compound or the pharmaceutically acceptable salt thereof to be administered to a subject in addition to said at least one second compound is a non-steroidal anti-inflammatory drug, preferably wherein said non-steroidal anti-inflammatory drug is Famprofazone, Clonixin, or a cyclooxygenase-2 inhibitor, more preferably wherein said cyclooxygenase-2 inhibitor is Tolfenamic acid, Celecoxib, or Meclofenamic acid.

In an even more preferable embodiment of this invention, said compound to be administered to a subject in addition to said at least one second compound is selected from Dicumarol, Tolfenamic acid, Famprofazone, Celecoxib, Benzbromarone, Diethylstilbestrol, Hexestrol, Lorglumide, Diflunisal, Iopanoic acid, Losartan, Tiratricol, Triclosan, Clonixin, Meclofenamic acid, Diclazuril, and Efavirenz, or a pharmaceutically acceptable salt thereof.

"Human-targeted drug", in the context of the present invention, shall refer to a compound intended for the use in humans. Preferably, the mechanism of action (MOA) of said drug is known and may affect a human cell intracellularly, extracellularly, or within the human cell membrane. The use of said human-targeted drug may suffer from the fact that it has side effects harming a human cell or organism. Examples of such human-targeted drugs include, without being limited thereto, antipsychotics, *anesthetics,* acid-reducing medications, chemotherapy drugs, and blood-pressure medications. Contrary, the term "antibiotic", in the context of the present invention, shall refer to a compound that is preferably microbiologically active, i.e. for use against pathogenic/undesired microbes.

Preferably, said at least one second compound is having an antibacterial effect on at least one bacterial species. More preferably, said at least one second compound is an antibiotic.

The term "antibiotic", as used herein, relates to a chemical substance, which at certain concentrations kills or prevents the growth of certain microorganisms, generally bacteria, although some antibiotics are also used for the treatment of infections caused by fungi or protozoa. Antibiotics are used in human, animal or horticultural medicine to treat infections caused by microorganisms. Antibiotics included in the present invention are, without limitation, aminoglycosides, polymyxins, oxazolidinones, strepotgramins, ansamycins, carbacefems, carbapenems, cephalosporins, glycopeptides, glycylcyclines, macrolides, monobactams, penicillins, polypeptides, quinolones, fluoroquinolones, sulphonamides, beta-lactams, tetracyclines, rifamycins, vancomycin, daptomycin, trimethoprim, novobiocin, arsphenamine, chloramphenicol, clindamycin, lincomycin, ethambutol, fosfomycin, fusidic acid, furazolidone, isoniazid, metronidazole, mupirocin, nitrofurantoin, platensimycin, pyrazinamide, quinupristin, dalfopristin, and combinations thereof.

In a particularly preferred embodiment, said at least one second compound is an antibiotic, wherein said antibiotic is preferably selected from a macrolide, a penicillin, a cephalosporin, a quinolone, a fluoroquinolone, a sulphonamide, a tetracycline, a monobactam, a carbapenem, an aminoglycoside, a rifamycin, a beta-lactam, an ansamycin, an oxazolidinone, a strepotgramin, a glycopeptide, a polypeptide, and an arsphenamine, or a pharmaceutically acceptable salt thereof.

In a further preferred embodiment, said at least one second compound is a macrolide antibiotic, preferably wherein said macrolide antibiotic is selected from erythromycin, azithromycin, clarithromycin, dirithromycin, and clindamycin, or a pharmaceutically acceptable salt thereof.

In another preferred embodiment, said at least one second compound is a tetracycline antibiotic, preferably wherein said tetracycline antibiotic is selected from doxycycline, minocycline, tigecyline, and lymecyclin, or a pharmaceutically acceptable salt thereof.

In yet another preferred embodiment, said at least one second compound is an aminoglycoside antibiotic, preferably wherein said antibiotic is selected streptomycin, dihydrostreptomycin, amikacin, apramycin, arbekacin, astromicin, bekanamycin, dibekacin, framycetin, gentamicin, hygromycin, isepamicin, kanamycin, neomycin, netilmicin, paromomycin, rhodostreptomycin, ribostamycin, sisomycin, spectinomycin, tobramycin, and verdamicin, or a pharmaceutically acceptable salt thereof.

In a further preferred embodiment, said at least one second compound is selected from erythromycin, azithromycin, clarithromycin, dirithromycin, clindamycin, doxycycline, minocycline, tigecyline, trimethoprim, pyocyanin, vancomycin, streptomycin, dihydrostreptomycin, amikacin, apramycin, arbekacin, astromicin, bekanamycin, dibekacin, framycetin, gentamicin, hygromycin, isepamicin, kanamycin, neomycin, netilmicin, paromomycin, rhodostreptomycin, ribostamycin, sisomycin, spectinomycin, tobramycin, verdamicin, polymixin, glycylcycline, carbapenem, carbacephem, chloramphenicol, clindamycin, lincomycin, daptomycin, novobiocin, clindamycin, ethambutol, fosfomycine, fusidic acid, furazolidone, isoniazid, linezolide, metronidazole, mupirocin, nitrofurantoin, platensimycine, pyrazinamide, quinupristine, benzalkonium, dalfopristine, rifampine, rifampicin, rifabutin, rifaximin, tinidazole, viomycin, and capreomycin, or a pharmaceutically acceptable salt thereof.

In a preferred embodiment of this invention, said compound is Dicumarol, Tolfenamic acid, Diethylstilbestrol, Hexestrol, Lorglumide, Benzbromarone, Famprofazone, or a pharmaceutically acceptable salt thereof, and said at least one second compound is an antibiotic, preferably wherein said at least one second compound is a macrolide.

In a further preferred embodiment of this invention, said compound is Dicumarol, Tolfenamic acid, Diethylstilbestrol, Hexestrol, Lorglumide, Benzbromarone, Famprofazone, or a pharmaceutically acceptable salt thereof, and said at least one second compound is a macrolide antibiotic, wherein said macrolide antibiotic is selected from as erythromycin, azithromycin, clarithromycin, dirithromycin, and clindamycin, or a pharmaceutically acceptable salt thereof.

Yet another preferred embodiment of this invention relates to said compound, wherein said compound is Dicumarol, or a pharmaceutically acceptable salt thereof, and said at least one second compound is a macrolide antibiotic, such as erythromycin, azithromycin, clarithromycin, dirithromycin, or clindamycin, preferably wherein said at least one second compound is erythromycin, or a pharmaceutically acceptable salt thereof.

In the context of this invention Dicumarol shall refer to 3,3'-Methylene-bis(4-hydroxycoumarin).

A further preferred embodiment of this invention relates to said compound, wherein said compound is Tolfenamic acid, or a pharmaceutically acceptable salt thereof, and said at least one second compound is a macrolide antibiotic, such as erythromycin, azithromycin, clarithromycin, dirithromycin, or clindamycin, preferably wherein said at least one second compound is erythromycin, or a pharmaceutically acceptable salt thereof.

An additionally preferred embodiment of this invention relates to said compound, wherein said compound is Diethylstilbestrol, or a pharmaceutically acceptable salt thereof, and said at least one second compound is a macrolide antibiotic, such as erythromycin, azithromycin, clarithromycin, dirithromycin, or clindamycin, preferably wherein said at least one second compound is erythromycin, or a pharmaceutically acceptable salt thereof.

Yet another preferred embodiment of this invention relates to said compound, wherein said compound is Hexestrol, or a pharmaceutically acceptable salt thereof, and said at least one second compound is a macrolide antibiotic, such as erythromycin, azithromycin, clarithromycin, dirithromycin, or clindamycin, preferably wherein said at least one second compound is erythromycin, or a pharmaceutically acceptable salt thereof.

A further preferred embodiment of this invention relates to said compound, wherein said compound is Lorglumide, or a pharmaceutically acceptable salt thereof, and said at least one second compound is a macrolide antibiotic, such as erythromycin, azithromycin, clarithromycin, dirithromycin, or clindamycin, preferably wherein said at least one second compound is erythromycin, or a pharmaceutically acceptable salt thereof.

In a further preferred embodiment of this invention, said compound is a Lorglumide sodium salt, and said at least one second compound is a macrolide antibiotic, such as erythromycin, azithromycin, clarithromycin, dirithromycin, or clindamycin, or a pharmaceutically acceptable salt thereof, preferably wherein said at least one second compound is erythromycin, or a pharmaceutically acceptable salt thereof.

In the context of this invention, Lorglumide sodium salt shall be the preferred pharmaceutically acceptable salt of Lorglumide.

Yet another preferred embodiment of this invention relates to said compound, wherein said compound is Benzbromarone, or a pharmaceutically acceptable salt thereof, and said at least one second compound is a macrolide antibiotic, such as erythromycin, azithromycin, clarithromycin, dirithromycin, or clindamycin, preferably wherein said at least one second compound is erythromycin, or a pharmaceutically acceptable salt thereof.

An additionally preferred embodiment of this invention relates to said compound, wherein said compound is Famprofazone, or a pharmaceutically acceptable salt thereof, and said at least one second compound is a macrolide antibiotic, such as erythromycin, azithromycin, clarithromycin, dirithromycin, or clindamycin, preferably wherein said at least one second compound is erythromycin, or a pharmaceutically acceptable salt thereof.

A further preferred embodiment relates to said compound, wherein said compound is selected from Diflunisal, Tolfenamic acid, Iopanoic acid, Tiratricol, Celecoxib, Losartan, Triclosan, Clonixin, Diethylstilbestrol, Meclofenamic acid, Diclazuril, and Efavirenz, or a pharmaceutically acceptable salt thereof, and said at least one second compound is an antibiotic, preferably wherein said at least one second compound is a tetracycline antibiotic.

In a further preferred embodiment of this invention, said at least one second compound is a tetracycline antibiotic, preferably wherein said at least one second compound is a tetracycline antibiotic selected from doxycycline, minocycline, tigecyline, and lymecyclin, or a pharmaceutically acceptable salt thereof.

An additionally preferred embodiment of this invention relates to said compound, wherein said compound is Diflunisal, or a pharmaceutically acceptable salt thereof, and said at least one second compound is a tetracycline antibiotic, such as doxycycline, minocycline, tigecyline, or lymecyclin, or a pharmaceutically acceptable salt thereof.

A further preferred embodiment of this invention relates to said compound, wherein said compound is Tolfenamic acid, or a pharmaceutically acceptable salt thereof, and said at least one second compound is a tetracycline antibiotic, such as doxycycline, minocycline, tigecyline, or lymecyclin, or a pharmaceutically acceptable salt thereof.

A further preferred embodiment of this invention relates to said compound, wherein said compound is Iopanoic acid, or a pharmaceutically acceptable salt thereof, and said at least one second compound is a tetracycline antibiotic, such as doxycycline, minocycline, tigecyline, or lymecyclin, or a pharmaceutically acceptable salt thereof.

Yet another preferred embodiment of this invention relates to said compound, wherein said compound is Tiratricol, or a pharmaceutically acceptable salt thereof, and said at least one second compound is a tetracycline antibiotic, such as doxycycline, minocycline, tigecyline, or lymecyclin, or a pharmaceutically acceptable salt thereof.

In the context of this invention, Tiratricol shall refer to 3,3',5-triiodothyroacetic acid.

Yet another preferred embodiment of this invention relates to said compound, wherein said compound is Celecoxib, or a pharmaceutically acceptable salt thereof, and said at least one second compound is a tetracycline antibiotic, such as doxycycline, minocycline, tigecyline, or lymecyclin, or a pharmaceutically acceptable salt thereof.

An additionally preferred embodiment of this invention relates to said compound, wherein said compound is Losartan, or a pharmaceutically acceptable salt thereof, and said at least one second compound is a tetracycline antibiotic, such as doxycycline, minocycline, tigecyline, or lymecyclin, or a pharmaceutically acceptable salt thereof.

In the context of this invention Losartan is preferably Losartan Potassium.

A further preferred embodiment of this invention relates to said compound, wherein said compound is Triclosan, or a pharmaceutically acceptable salt thereof, and said at least one second compound is a tetracycline antibiotic, such as doxycycline, minocycline, tigecyline, or lymecyclin, or a pharmaceutically acceptable salt thereof.

Yet another preferred embodiment of this invention relates to said compound, wherein said compound is Clonixin, or a pharmaceutically acceptable salt thereof, and said at least one second compound is a tetracycline antibiotic, such as doxycycline, minocycline, tigecyline, or lymecyclin, or a pharmaceutically acceptable salt thereof.

In the context of this invention Clonixin can also be Clonixin Lysinate.

An additionally preferred embodiment of this invention relates to said compound, wherein said compound is Diethylstilbestrol, or a pharmaceutically acceptable salt thereof, and said at least one second compound is a tetracycline antibiotic, such as doxycycline, minocycline, tigecyline, or lymecyclin, or a pharmaceutically acceptable salt thereof.

A further preferred embodiment of this invention relates to said compound, wherein said compound is Meclofenamic acid, or a pharmaceutically acceptable salt thereof, and said at least one second compound is a tetracycline antibiotic, such as doxycycline, minocycline, tigecyline, or lymecyclin, or a pharmaceutically acceptable salt thereof.

Yet another further preferred embodiment of this invention relates to said compound, wherein said compound is Meclofenamic acid sodium salt monohydrate, or a pharmaceutically acceptable salt thereof, and said at least one second compound is a tetracycline antibiotic, such as doxycycline, minocycline, tigecyline, lymecyclin, or a pharmaceutically acceptable salt thereof.

In the context of this invention, a Meclofenamic acid sodium salt monohydrate is the preferred pharmaceutically acceptable salt of Meclofenamic acid.

An additionally preferred embodiment of this invention relates to said compound, wherein said compound is Diclazuril, or a pharmaceutically acceptable salt thereof, and said at least one second compound is a tetracycline antibiotic, such as doxycycline, minocycline, tigecyline, or lymecyclin, or a pharmaceutically acceptable salt thereof.

A further preferred embodiment of this invention relates to said compound, wherein said compound is Efavirenz, or a pharmaceutically acceptable salt thereof, and said at least one second compound is a tetracycline antibiotic, such as doxycycline, minocycline, tigecyline, or lymecyclin, or a pharmaceutically acceptable salt thereof.

In a further preferred embodiment, said at least one first bacterium is a Gram-positive bacterium or a Gram-negative bacterium, preferably wherein said at least one first bacterium is a member of a genus selected from *Bacteroides, Eubacterium, Faecalibacterium, Odoribacter, Prevotella, Parabacteroides, Ruminococcus, Roseburia, Bifidobacterium, Coprococcus, Dorea, Blautia, Clostridium, Escherichia, Streptococcus, Collinsella, Bilophila, Akkermansia, Veillonella, Haemophilus, Desulfovibrio, Fusobacterium, Lactobacillus, Alistipes,* and *Butyrivibrio.*

Additionally preferred is that said at least one first bacterium is at least one bacterial species selected from *Bacteroides vulgatus, Bacteroides uniformis, Bacteroides caccae, Bacteroides fragilis NT, Bacteroides ovatus, Bacteroides thetaiotaomicron, Eubacterium rectale, Faecalibacterium prausnitzii, Odoribacter splanchnicus, Prevotella copri, Parabacteroides distasonis, Ruminococcus gnavus, Ruminococcus torques, Ruminococcus bromii, Ruminococcus obeum, Roseburia hominis, Roseburia intestinalis, Parabacteroides merdae, Bifidobacterium adolescentis, Bifidobacterium longum, Coprococcus comes, Dorea formicigenerans, Blautia hansenii, Blautia obeum, Clostridium bolteae, Clostridium leptum, Clostridium ramosum, Eggerthella lenta, Streptococcus parasanguinis, Streptococcus salivarius, Collinsella aerofaciens, Bilophila wadsworthia, Escherichia coli, Akkermansia muciniphila, Veillonella parvula, Haemophilus parainfluenzae, Desulfovibrio piger, Fusobacterium nucleatum, Clostridium perfingens, Clostridium saccharolyticum, Lactobacillus paracasei, Bacteroides clarus, Bacteroides coprocola, Bacteroides dorei, Bacteroides eggerthii, Bacteroides xylanisolvens, Bacteroides stercoris, Eubacterium eligens, Eubacterium siraeum, Alistipes putredinis, Alistipes shahii,* and *Butyrivibrio crossotus.*

Then, a further preferred embodiment relates to said at least one second bacterium, wherein said at least one second bacterium is a Gram-positive bacterium or a Gram-negative bacterium, and wherein said at least one second bacterium is a member of a genus selected from *Enterococcus, Staphylococcus, Enterobacter, Streptococcus, Pseudomonas, Escherichia, Salmonella, Helicobacter, Neisseria, Campylobacter, Chlamydia, Clostridia, Citrobacter, Vibrio, Treponema, Mycobacterium, Klebsiella, Actinomyces, Bacteroides, Bordetella, Borrelia, Brucella, Corynebacteria, Diplococcus, Fusobacterium, Leptospira, Listeria, Pasteurella, Proteus, Rickettsia, Shigella, Yersinia, Parabacteroides, Odoribacter, Faecalibacteria, Collinsella, Eggerthella, Lactonifactor, Pediococcus, Leuconostoc, Lactococcus, Roseburia, Coliform, Bacillus, Franscicella, Acinetobacter, Legionella, Actinobacillus, Coxiella, Kingella kingae, Haemophilus,* and combinations thereof, optionally wherein said second bacterium is an antibiotic-resistant bacterium and/or a multi-drug resistant bacterium.

In a further preferred embodiment of this invention, said at least one second bacterium is a pathogenic bacterium.

In the context of this invention, at least one bacterial species can be 1 bacterial species, or 2, 3, 4, 5, 6, 7, 8, 9, 10, 11, 12, 13, 14, 15, 16,17, 18, 19, 20, 21, 22, 23, 24, 25, 26, 27, 28, 29, 30, 31, 32, 33, 34, 35, 36, 37, 38, 39, 40, or any other number of bacterial species.

In a preferred embodiment, said compound is antagonizing said antibacterial effect of said at least one second compound on 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 bacterial species, or any number of bacterial species.

The compounds of this invention can generally be used in a wide variety of medical applications, in particular in the prevention and/or treatment of bacterial infections and/or dysbiosis. Preferred is a compound of this invention, wherein said at least one second compound is for use in the prevention and/or treatment of a bacterial infection, preferably wherein said bacterial infection is selected from an infection of the gastrointestinal tract, an infection of the urogenital tract, an infection of the upper and lower respiratory tract, rhinitis, tonsillitis, pharyngitis, bronchitis, pneumonia, an infection of the inner organs, nephritis, hepatitis, peritonitis, endocarditis, meningitis, osteomyelitis, an infection of the eyes, an infection of the ears, a cutaneous infection, a subcutaneous infection, an infection after burn, diarrhea, colitis, pseudomembranous colitis, a skin disorder, toxic shock syndrome, bacteremia, sepsis, pelvic inflammatory disease, an infection of the central nervous system, wound infection, intra-abdominal infection, intravascular infection, bone infection, joint infection, acute bacterial otitis media, pyelonephritis, deep-seated abscess, and tuberculosis.

The term "infection", as used in the context of the present invention, relates to the presence of bacteria, viruses, fungi, protozoa or other microorganisms, in or on a subject as well as the invasion by bacteria, viruses, fungi, protozoa or other microorganisms. The invasion includes undesired proliferation of pathogenic microbes in a host organism. More generally, a microbial infection can be any situation, in which the presence of a microbial population(s) is damaging to a host, such as a host animal. Thus, a microbial infection exists when excessive microorganisms are present in or on a mammal's body, or when the effects of the presence of a microbial population(s) is damaging the cells or other tissue of a mammal. Thus, the inhibition of the growth of such invading microorganisms results in a benefit to the subject that is infected by the microbial population(s). Examples of bacterial infections are urinary tract infection (UTI), kidney infections (pyelonephritis), gynecological and obstetrical infections, respiratory tract infection (RTI), acute exacerbation of chronic bronchitis (AECB), community-acquired pneumonia (CAP), hospital-acquired pneumonia (HAP), ventilator associated pneumonia (VAP), intra-abdominal pneumonia (IAI), acute otitis media, acute sinusitis, sepsis, catheter-related sepsis, chancroid, chlamydia, skin infections, and bacteremia.

The term "treating" as used in the context of this invention means stabilizing or reducing an adverse symptom associated with a condition; reducing the severity of a disease symptom; slowing the rate of the progression of a disease; inhibiting or stabilizing the progression of a disease condition; or changing a metric that is associated with the disease state in a desirable way.

A further preferred embodiment relates to a compound of this invention, wherein said compound is in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, room spray, or combinations thereof.

The compounds of this invention are preferably administered to a subject.

In the context of the present invention, the term "subject", as used in certain embodiments, preferably refers to a mammal, such as a mouse, rat, guinea pig, rabbit, cat, dog, monkey, or preferably a human. The subject of the invention may be at danger of suffering from a disease, such as an infection, for example a bacterial infection, a viral infection, a fungal infection, or a parasitic infection. A more detailed description of medical indications relevant in context of the invention is provided herein elsewhere.

As used herein, the term "patient" refers to any animal (e.g., a mammal), including, but not limited to, humans, non-human primates, rodents, and the like, which is to be the recipient of a particular treatment or diagnostic method. The term "patient" preferably refers to a human, for example a human patient, for whom diagnosis, prognosis, or therapy is desired. Typically, the terms "subject" and "patient" are used interchangeably herein in reference to a human subject. As used herein, the term "subject suspected of having a disease" refers to a subject that presents one or more symptoms indicative of a disease. A subject suspected of having a disease may also have one or more risk factors. The present invention is also applicable as follow-up care for monitoring a subject for a reoccurrence of the disease.

In a particularly preferred embodiment, the subject is a mammal, such as a mouse, a rat, a guinea pig, a rabbit, a cat, a dog, a monkey, or a human, preferably a human, such as a human patient, more preferably a human patient suffering from a disease, such as a bacterial infection, and in need of a therapy, most preferably a human patient suffering from a bacterial infection and in need of a therapy.

In a further preferred embodiment, the subject is a mammal, such as a mouse, rat, guinea pig, rabbit, cat, dog, monkey, or preferably a human, such as a human patient, optionally wherein said compound is administered to the subject in a therapeutically effective amount, thereby preventing and/or treating a disease in said subject, and/or preventing a modification of the microbiome of said subject.

Yet another aspect of this invention relates to a pharmaceutical composition, comprising
i) at least one compound according to this invention; and
ii) a pharmaceutically acceptable additive, carrier, diluent, solvent, filter, lubricant, excipient, binder, and/or stabilizer.

In the context of this invention, the term "pharmaceutical composition" shall refer to a preparation which is in such form as to permit the biological activity of an active ingredient contained therein to be effective, and which contains no additional components which are unacceptably toxic to a subject to which the composition would be administered. A pharmaceutical composition of the present invention can be administered by a variety of methods known in the art. As will be appreciated by the skilled artisan, the route and/or mode of administration will vary depending upon the desired results. Pharmaceutically acceptable diluents include, for example, saline and aqueous buffer solutions. A "pharmaceutically acceptable carrier" refers to an ingredient in a pharmaceutical formulation, other than an active ingredient, which is nontoxic to a subject. Pharmaceutically acceptable carriers include any and all solvents, dispersion media, coatings, antibacterial and antifungal agents, isotonic and absorption delaying agents, and the like that are physiologically compatible. The carrier can be suitable for intravenous, intramuscular, subcutaneous, parenteral, spinal, epidermal, or any other commonly used route of administration (e.g. by injection or infusion).

The pharmaceutical compositions according to this invention may also contain adjuvants such as preservatives, wetting agents, emulsifying agents and dispersing agents. Prevention of presence of microorganisms may be ensured both by sterilization procedures, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents, which delay absorption, such as aluminum monostearate and gelatin.

The pharmaceutical compositions of this invention must be sterile and fluid to the extent that the composition is deliverable by syringe. In addition to water, in one embodiment the carrier is an isotonic buffered saline solution. Proper fluidity can be maintained, for example, by use of coating such as lecithin, by maintenance of required particle size in the case of dispersion and by use of surfactants. In many cases, it is preferable to include isotonic agents, for example, sugars, polyalcohols, such as mannitol or sorbitol, and sodium chloride in the composition.

A further aspect of this invention relates to a pharmaceutical composition, comprising:
(i) at least one first compound, or a pharmaceutically acceptable salt thereof, wherein said at least one first compound is capable of antagonizing an antibacterial effect of at least one second compound in a first bacterium, and wherein said at least one first compound is not capable of antagonizing an antibacterial effect of said at least one second compound in a second bacterium;
(ii) at least one second compound, or a pharmaceutically acceptable salt thereof, and
(iii) optionally, a pharmaceutically acceptable additive, carrier, diluent, solvent, filter, lubricant, excipient, binder, and/or stabilizer.

Said at least one first compound (i) of the pharmaceutical composition of this invention can be any compound. In a preferred embodiment, said at least one first compound (i) of the pharmaceutical composition of this invention is selected from an anticoagulant drug, a non-steroidal anti-inflammatory drug, an uricosuric, a nonsteroidal estrogen compound, an antagonist of the cholecystokinin CCKA receptor, a salicylic acid derivative, a cyclooxygenase-2 inhibitor, a cholecystographic agent, an angiotensin II receptor blocker, a thyroid hormone analogue, an antibacterial agent, an antifungal agent, or a pharmaceutically acceptable salt thereof.

In a further preferred embodiment, said at least one second compound (ii) is an antibiotic, preferably wherein said antibiotic is selected from a macrolide, a penicillin, a cephalosporin, a quinolone, a fluoroquinolone, a sulphonamide, a tetracycline, a monobactam, a carbapnem, an aminoglycoside, a rifamycin, a beta-lactam, an ansamycin, an oxazolidinone, a strepotgramin, a glycopeptide, a polypeptide, and an arsphenamine, or a pharmaceutically acceptable salt thereof, more preferably wherein said antibiotic is selected from erythromycin, azithromycin, clarithromycin, dirithromycin, clindamycin, doxycycline, minocycline, tigecyline, trimethoprim, pyocyanin, vancomycin, streptomycin, dihydrostreptomycin, amikacin, apramycin, arbekacin, astromicin, bekanamycin, dibekacin, framycetin, gentamicin, hygromycin, isepamicin, kanamycin, neomycin, netilmicin, paromomycin, rhodostreptomycin, ribostamycin, sisomycin, spectinomycin, tobramycin, verdamicin, polymixin, glycylcycline, carbapenem, carbacephem, chloramphenicol, clindamycin, lincomycin, daptomycin, novobiocin, clindamycin, ethambutol, fosfomycine, fusidic acid, furazolidone, isoniazid, linezolide, metronidazole, mupirocin, nitrofurantoin, platensimycine, pyrazinamide, quinupristine, benzalkonium, dalfopristine, rifampine, rifampicin, rifabutin, rifaximin, tinidazole, viomycin, and capreomycin, or a pharmaceutically acceptable salt thereof.

In a preferred embodiment, said at least one first compound (i) or the pharmaceutically acceptable salt thereof of the pharmaceutical composition of this invention is selected from an anticoagulant drug, such as Dicumarol, a non-steroidal anti-inflammatory drug, such as Famprofazone, Tolfenamic acid, Celecoxib, Clonixin, or Meclofenamic acid, an Uricosuric, such as Benzbromarone, a nonsteroidal estrogen compound, such as Diethylstilbestrol or Hexestrol, an antagonist of the Cholecystokinin CCKA receptor, such as Lorglumide, a salicylic acid derivative, such as Diflunisal, a cholecystographic agent, such as Iopanoic acid, an angiotensin II receptor blocker, such as Losartan, a thyroid hormone analogue, such as Tiratricol, an antibacterial, antifungal and/or antibacterial agent, such as Triclosan or Diclazuril, a reverse transcriptase inhibitor, such as Efavirenz, a Ca-channel inhibitor, a human-targeted drug, a food additive, or a pharmaceutically acceptable salt thereof.

In yet another preferred embodiment said at least one first compound (i) or the pharmaceutically acceptable salt thereof of the pharmaceutical composition of this invention is a non-steroidal anti-inflammatory drug, preferably wherein said non-steroidal anti-inflammatory drug is Famprofazone, Clonixin, or a cyclooxygenase-2 inhibitor, more preferably wherein said cyclooxygenase-2 inhibitor is Tolfenamic acid, Celecoxib, or Meclofenamic acid.

In an additionally preferred embodiment, said at least one first compound (i) of the pharmaceutical composition of this invention is selected from Dicumarol, Tolfenamic acid, Famprofazone, Celecoxib, Benzbromarone, Diethylstilbestrol, Hexestrol, Lorglumide, Diflunisal, Iopanoic acid, Losartan, Tiratricol, Triclosan, Clonixin, Meclofenamic acid, Diclazuril, and Efavirenz, or a pharmaceutically acceptable salt thereof.

A further preferred embodiment relates to a pharmaceutical composition of this invention, wherein said at least one first compound (i) is Dicumarol, Tolfenamic acid, Diethylstilbestrol, Hexestrol, Lorglumide, Benzbromarone, Famprofazone, or a pharmaceutically acceptable salt thereof, and said at least one second compound is an antibiotic, preferably wherein said at least one second compound (ii) is a macrolide antibiotic, such as erythromycin, azithromycin, clarithromycin, dirithromycin, or clindamycin, preferably wherein said at least one second compound is erythromycin, or a pharmaceutically acceptable salt thereof.

Yet another preferred embodiment of this invention relates to said pharmaceutical composition, wherein said at least one first compound (i) is Dicumarol, or a pharmaceutically acceptable salt thereof, and said at least one second compound (ii) is a macrolide antibiotic, such as erythromycin, azithromycin, clarithromycin, dirithromycin, or clindamycin, preferably wherein said at least one second compound is erythromycin, or a pharmaceutically acceptable salt thereof.

In the context of this invention Dicumarol shall refer to 3,3'-Methylene-bis(4-hydroxycoumarin).

A further preferred embodiment of this invention relates to said pharmaceutical composition, wherein said at least one first compound (i) is Tolfenamic acid, or a pharmaceutically acceptable salt thereof, and said at least one second compound (ii) is a macrolide antibiotic, such as erythromycin, azithromycin, clarithromycin, dirithromycin, or clindamycin, preferably wherein said at least one second compound is erythromycin, or a pharmaceutically acceptable salt thereof.

An additionally preferred embodiment of this invention relates to said pharmaceutical composition, wherein said at least one first compound (i) is Diethylstilbestrol, or a pharmaceutically acceptable salt thereof, and said at least one second compound (ii) is a macrolide antibiotic, such as erythromycin, azithromycin, clarithromycin, dirithromycin, or clindamycin, preferably wherein said at least one second compound is erythromycin, or a pharmaceutically acceptable salt thereof.

Yet another preferred embodiment of this invention relates to said pharmaceutical composition, wherein said at least one first compound (i) is Hexestrol, or a pharmaceutically acceptable salt thereof, and said at least one second compound (ii) is a macrolide antibiotic, such as erythromycin, azithromycin, clarithromycin, dirithromycin, or clindamycin, preferably wherein said at least one second compound is erythromycin, or a pharmaceutically acceptable salt thereof.

A further preferred embodiment of this invention relates to said pharmaceutical composition, wherein said at least one first compound (i) is Lorglumide, or a pharmaceutically acceptable salt thereof, and said at least one second compound (ii) is a macrolide antibiotic, such as erythromycin, azithromycin, clarithromycin, dirithromycin, or clindamycin, preferably wherein said at least one second compound is erythromycin, or a pharmaceutically acceptable salt thereof.

Yet another preferred embodiment of this invention relates to said pharmaceutical composition, wherein said at least one first compound (i) is a Lorglumide sodium salt, or a pharmaceutically acceptable salt thereof, and said at least one second compound (ii) is a macrolide antibiotic, such as erythromycin, azithromycin, clarithromycin, dirithromycin, or clindamycin, preferably wherein said at least one second compound is erythromycin.

In the context of this invention, Lorglumide sodium salt is the preferred pharmaceutically acceptable salt of Lorglumide.

Yet another preferred embodiment of this invention relates to said pharmaceutical composition, wherein said at least one first compound (i) is Benzbromarone, or a pharmaceutically acceptable salt thereof, and said at least one second compound (ii) a macrolide antibiotic, such as erythromycin, azithromycin, clarithromycin, dirithromycin, or clindamycin, preferably wherein said at least one second compound is erythromycin, or a pharmaceutically acceptable salt thereof.

An additionally preferred embodiment of this invention relates to said pharmaceutical composition, wherein said at least one first compound (i) is Famprofazone, or a pharmaceutically acceptable salt thereof, and said at least one second compound (ii) is a macrolide antibiotic, such as erythromycin, azithromycin, clarithromycin, dirithromycin, or clindamycin, preferably wherein said at least one second compound is erythromycin, or a pharmaceutically acceptable salt thereof.

A further preferred embodiment relates to said pharmaceutical composition, wherein said at least one first compound (i) is selected from Diflunisal, Tolfenamic acid, Iopanoic acid, Tiratricol, Celecoxib, Losartan, Triclosan, Clonixin, Diethylstilbestrol, Meclofenamic acid, Diclazuril, and Efavirenz, or a pharmaceutically acceptable salt thereof, and said at least one second compound (ii) is an antibiotic, preferably wherein said at least one second compound is a tetracycline antibiotic, such as doxycycline, minocycline, tigecyline, or lymecyclin, or a pharmaceutically acceptable salt thereof.

In a further preferred embodiment of this invention, said at least one second compound (ii) is a tetracycline antibiotic, preferably wherein said at least one second compound is a tetracycline antibiotic selected from doxycycline, minocycline, tigecyline, and lymecyclin, or a pharmaceutically acceptable salt thereof.

An additionally preferred embodiment of this invention relates to said pharmaceutical composition, wherein said at least one first compound (i) is Diflunisal, or a pharmaceutically acceptable salt thereof, and said at least one second compound (ii) is a tetracycline antibiotic, such as doxycycline, minocycline, tigecyline, or lymecyclin, or a pharmaceutically acceptable salt thereof.

A further preferred embodiment of this invention relates to said pharmaceutical composition, wherein said at least one first compound (i) is Tolfenamic acid, or a pharmaceutically acceptable salt thereof, and said at least one second compound (ii) is a tetracycline antibiotic, such as doxycycline, minocycline, tigecyline, or lymecyclin, or a pharmaceutically acceptable salt thereof.

A further preferred embodiment of this invention relates to said pharmaceutical composition, wherein said at least one first compound (i) is Iopanoic acid, or a pharmaceutically acceptable salt thereof, and said at least one second compound (ii) is a tetracycline antibiotic, such as doxycycline, minocycline, tigecyline, or lymecyclin, or a pharmaceutically acceptable salt thereof.

Yet another preferred embodiment of this invention relates to said pharmaceutical composition, wherein said at least one first compound (i) is Tiratricol, or a pharmaceutically acceptable salt thereof, and said at least one second compound (ii) is a tetracycline antibiotic, such as doxycycline, minocycline, tigecyline, or lymecyclin, or a pharmaceutically acceptable salt thereof.

In the context of this invention, Tiratricol shall refer to 3,3',5-triiodothyroacetic acid.

Yet another preferred embodiment of this invention relates to said pharmaceutical composition, wherein said at least one first compound (i) is Celecoxib, or a pharmaceutically acceptable salt thereof, and said at least one second compound (ii) is a tetracycline antibiotic, such as doxycycline, minocycline, tigecyline, or lymecyclin, or a pharmaceutically acceptable salt thereof.

An additionally preferred embodiment of this invention relates to said pharmaceutical composition, wherein said at least one first compound (i) is Losartan, or a pharmaceutically acceptable salt thereof, and said at least one second compound (ii) is a tetracycline antibiotic, such as doxycycline, minocycline, tigecyline, or lymecyclin, or a pharmaceutically acceptable salt thereof.

In the context of this invention Losartan is preferably Losartan Potassium.

A further preferred embodiment of this invention relates to said pharmaceutical composition, wherein said at least one first compound (i) is Triclosan, or a pharmaceutically acceptable salt thereof, and said at least one second compound (ii) is a tetracycline antibiotic, such as doxycycline, minocycline, tigecyline, or lymecyclin, or a pharmaceutically acceptable salt thereof.

Yet another preferred embodiment of this invention relates to said pharmaceutical composition, wherein said at least one first compound (i) is Clonixin, or a pharmaceutically acceptable salt thereof, and said at least one second compound (ii) is a tetracycline antibiotic, such as doxycycline, minocycline, tigecyline, or lymecyclin, or a pharmaceutically acceptable salt thereof.

In the context of this invention Clonixin can also be Clonixin Lysinate. In a further preferred embodiment, said at least one first compound (i) of said pharmaceutical composition is Clonixin Lysinate, and said at least one second compound (ii) is a tetracycline antibiotic, such as doxycycline, minocycline, tigecyline, or lymecyclin, or a pharmaceutically acceptable salt thereof.

An additionally preferred embodiment of this invention relates to said pharmaceutical composition, wherein said at least one first compound (i) is Diethylstilbestrol, or a pharmaceutically acceptable salt thereof, and said at least one second compound (ii) is a tetracycline antibiotic, such as doxycycline, minocycline, tigecyline, or lymecyclin, or a pharmaceutically acceptable salt thereof.

A further preferred embodiment of this invention relates to said pharmaceutical composition, wherein said at least one first compound (i) is Meclofenamic acid, or a pharmaceutically acceptable salt thereof, and said at least one second compound (ii) is a tetracycline antibiotic, such as doxycycline, minocycline, tigecyline, or lymecyclin, or a pharmaceutically acceptable salt thereof.

Another further preferred embodiment of this invention relates to said pharmaceutical composition, wherein said at least one first compound (i) is Meclofenamic acid sodium salt monohydrate, and said at least one second compound (ii) is a tetracycline antibiotic, such as doxycycline, minocycline, tigecyline, or lymecyclin, or a pharmaceutically acceptable salt thereof.

In the context of this invention, a Meclofenamic acid sodium salt monohydrate is the preferred pharmaceutically acceptable salt of Meclofenamic acid.

An additionally preferred embodiment of this invention relates to said pharmaceutical composition, wherein said at least one first compound (i) is Diclazuril, or a pharmaceutically acceptable salt thereof, and said at least one second compound (ii) is a tetracycline antibiotic, such as doxycycline, minocycline, tigecyline, or lymecyclin, or a pharmaceutically acceptable salt thereof.

A further preferred embodiment of this invention relates to said pharmaceutical composition, wherein said at least one first compound (i) is Efavirenz, or a pharmaceutically acceptable salt thereof, and said at least one second compound (ii) is a tetracycline antibiotic, such as doxycycline, minocycline, tigecyline, or lymecyclin, or a pharmaceutically acceptable salt thereof.

Yet another preferred embodiment relates to the pharmaceutical composition of this invention, wherein said at least one first bacterium is a Gram-positive bacterium or a Gram-negative bacterium, preferably wherein said at least one first bacterium is a member of a genus selected from *Bacteroides, Eubacterium, Faecalibacterium, Odoribacter, Prevotella, Parabacteroides, Ruminococcus, Roseburia, Bifidobacterium, Coprococcus, Dorea, Blautia, Clostridium, Escherichia, Streptococcus, Collinsella, Bilophila, Akkermansia, Veillonella, Haemophilus, Desulfovibrio, Fusobacterium, Lactobacillus, Alistipes,* and *Butyrivibrio.*

Yet another preferred embodiment relates to a pharmaceutical composition of this invention, wherein said at least one first bacterium is at least one bacterial species selected from *Bacteroides vulgatus, Bacteroides uniformis, Bacteroides caccae, Bacteroides fragilis NT, Bacteroides ovatus, Bacteroides thetaiotaomicron, Eubacterium rectale, Faecalibacterium prausnitzii, Odoribacter splanchnicus, Prevotella copri, Parabacteroides distasonis, Ruminococcus gnavus, Ruminococcus torques, Ruminococcus bromii, Ruminococcus obeum, Roseburia hominis, Roseburia intestinalis, Parabacteroides merdae, Bifidobacterium adolescentis, Bifidobacterium longum, Coprococcus comes, Dorea formicigenerans, Blautia hansenii, Blautia obeum, Clostridium bolteae, Clostridium leptum, Clostridium ramosum, Eggerthella lenta, Streptococcus parasanguinis, Streptococcus salivarius, Collinsella aerofaciens, Bilophila wadsworthia, Escherichia coli, Akkermansia muciniphila, Veillonella parvula, Haemophilus parainfluenzae, Desulfovibrio piger, Fusobacterium nucleatum, Clostridium perfingens, Clostridium saccharolyticum, Lactobacillus paracasei, Bacteroides clarus, Bacteroides coprocola, Bacteroides dorei, Bacteroides eggerthiiBacteroides xylanisolvens, Bacteroides stercoris, Eubacterium eligens, Eubacterium siraeum, Alistipes putredinis, Alistipes shahii,* and *Butyrivibrio crossotus.*

A further preferred embodiment relates to the pharmaceutical composition of this invention, wherein said at least one second bacterium is a Gram-positive bacterium or a Gram-negative bacterium, and wherein said at least one second bacterium is a member of a genus selected from *Enterococcus, Staphylococcus, Enterobacter, Streptococcus, Pseudomonas, Escherichia, Salmonella, Helicobacter, Neisseria, Campylobacter, Chlamydia, Clostridia, Citrobacter, Vibrio, Treponema, Mycobacterium, Klebsiella, Actinomyces, Bacteroides, Bordetella, Borrelia, Brucella, Corynebacteria, Diplococcus, Fusobacterium, Leptospira, Listeria, Pasteurella, Proteus, Rickettsia, Shigella, Yersinia, Parabacteroides, Odoribacter, Faecalibacteria, Collinsella, Eggerthella, Lactonifactor, Pediococcus, Leuconostoc, Lactococcus, Roseburia, Coliform, Bacillus, Franscicella, Acinetobacter, Legionella, Actinobacillus, Coxiella, Kingella kingae, Haemophilus,* and combinations thereof, optionally wherein said at least one second bacterium is an antibiotic-resistant bacterium and/or a multi-drug resistant bacterium.

In a further preferred embodiment, said at least one second bacterium is a pathogenic bacterium.

The pharmaceutical composition of this invention can generally be used in a wide variety of medical applications, in particular in the prevention and/or treatment of bacterial infections and/or dysbiosis. Preferred is a pharmaceutical composition, wherein said at least one second compound (ii) is for use in the prevention and/or treatment of a bacterial infection, wherein said bacterial infection is preferably selected from an infection of the gastrointestinal tract, an infection of the urogenital tract, an infection of the upper and lower respiratory tract, rhinitis, tonsillitis, pharyngitis, bronchitis, pneumonia, an infection of the inner organs, nephritis, hepatitis, peritonitis, endocarditis, meningitis, osteomyelitis, an infection of the eyes, an infection of the ears, a cutaneous infection, a subcutaneous infection, an infection after burn, diarrhea, colitis, pseudomembranous colitis, a skin disorder, toxic shock syndrome, bacteremia, sepsis, pelvic inflammatory disease, an infection of the central nervous system, wound infection, intra-abdominal infection, intravascular infection, bone infection, joint infection, acute bacterial otitis media, pyelonephritis, deep-seated abscess, and tuberculosis.

An additionally preferred embodiment pertains to the afore-described pharmaceutical composition according to this invention, wherein said components (i) and (ii) of said pharmaceutical composition are administered to a subject simultaneously, separately, or sequentially.

Yet another preferred embodiment relates to a pharmaceutical composition of this invention, wherein said pharmaceutical composition is in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, or room spray.

A further preferred embodiment relates to the pharmaceutical composition of this invention, wherein said pharmaceutical composition is administered to a subject. In a particularly preferred embodiment, said subject is a mammal, such as a mouse, rat, guinea pig, rabbit, cat, dog, monkey, or preferably a human, such as a human patient, optionally wherein said pharmaceutical composition is administered to said subject in a therapeutically effective amount, thereby preventing and/or treating a disease in said subject, and/or preventing a modification of the microbiome of said subject.

Yet another preferred embodiment relates to the compound of this invention, wherein said compound is administered to the subject by any suitable means including oral, intravenous, arterial infusion, intraperitoneal, parenteral, enteral, topical, intramuscular, subcutaneous, surgical, topical, cutaneous, subcutaneous, or any other clinically approved way of administration.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection, and infusion. Regardless of the route of administration selected, the compounds and pharmaceutical compositions of the present invention, which may be used in a suitable hydrated form, are formulated into pharmaceutically acceptable dosage forms by conventional methods known to those skilled in the art. Actual dosage levels of the active ingredients in the pharmaceutical compositions of the present invention may be varied so as to obtain an amount of the active ingredient, which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and other factors well known in the medical arts.

The dosage regimen of the compounds of this invention and/or the pharmaceutical composition of this invention will be determined by the attending physician and clinical factors. As is well known in the medical arts, dosages for any one patient depend upon many factors, including the patient's size, body surface area, age, the particular compound to be administered, sex, time and route of administration, general health, and other drugs or compounds being administered concurrently. A typical dose can be, for example, in the range of 0.001 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment.

The skilled artisan will understand that numerous methods may be used to determine the dosage regimen of the compounds of this invention and/or the pharmaceutical compositions.

The compounds and pharmaceutical compositions of the invention may be administered locally or systemically. Administration will preferably be parenterally; the compounds and pharmaceutical compositions may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils, such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present, such as, for example, antimicrobials, anti-oxidants, chelating agents, and inert gases and the like. Furthermore, the compounds and pharmaceutical compositions of the invention may comprise further agents such as interleukins or interferons depending on the intended use of the compounds and pharmaceutical compositions.

An additional aspect of this invention pertains to a compound according to this invention, and/or a pharmaceutical composition of this invention, for use in medicine.

A further aspect of the present invention relates to a compound according to this invention, or a pharmaceutical composition according to this invention, for use in the prevention and/or treatment of a disease in a subject, and/or in the prevention of a modification of the microbiome of said subject, and/or in the manufacture of a medicament for the treatment of a disease.

In yet another preferred embodiment of this invention, said compound according to this invention, or said pharmaceutical composition according to this invention, is for use in the prevention of a modification of the microbiome of said subject, and/or in the manufacture of a medicament for the prevention of a modification of the microbiome of said subject, and/or for the treatment of dysbiosis, preferably wherein said microbiome comprises commensal bacterial species.

A further preferred embodiment relates to the afore-described compound for use, or to the afore-described pharmaceutical composition for use, wherein said compound is administered to a subject, thereby modifying the growth of bacterial cells in said subject, wherein said modifying results in the prevention and/or treatment of a disease in said subject, and/or in the prevention of a modification of the microbiome of said subject.

Yet another aspect of this invention relates to a method for preventing an adverse effect on a gut microbiome using a compound according to this invention, and/or using a pharmaceutical composition of this invention, the method comprising administering to a subject an effective amount of a compound according to this invention, and/or a pharmaceutical composition of this invention, thereby treating the disease in the subject, optionally wherein said method prevents of a modification of the microbiome of said subject.

Another preferred embodiment of this invention pertains to said method for preventing an adverse effect on a gut microbiome using a compound according to this invention, and/or using a pharmaceutical composition of this invention, the method comprising administering to a subject an effective amount of a compound according to this invention, and/or a pharmaceutical composition of this invention, thereby treating the disease in the subject, wherein said method prevents of a modification of the microbiome of said subject, preferably wherein said microbiome is the commensal microbiome.

Yet another aspect of this invention pertains to a compound according to this invention, and/or a pharmaceutical composition of this invention, for use in the treatment of a disease, such as an infectious disease or dysbiosis, or for use in the manufacture of a medicament against a disease, such as an infectious disease or dysbiosis.

A further aspect of this invention relates to a kit, comprising:
a) a container comprising a compound according to this invention, and/or comprising a pharmaceutical composition of this invention, in solution or in lyophilized form;
b) optionally, a second container containing a diluent or reconstituting solution for the lyophilized formulation, and
d) optionally, instructions for (i) use of the solution or (ii) reconstitution and/or use of the lyophilized formulation.

An additional embodiment of this invention relates to the afore-mentioned kit, wherein said kit further comprises one or more of (iii) a buffer, (iv) a diluent, (v) a filter, (vi) a needle, or (v) a syringe.

Yet another aspect of this invention relates to a method for killing bacterial cells in a subject, the method comprising administering to the subject an effective amount of a compound according to this invention, and/or a pharmaceutical composition of this invention.

A further aspect of this invention pertains to the use of an effective amount of a compound according to this invention, and/or a pharmaceutical composition of this invention for killing bacterial cells in a subject.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B, and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:

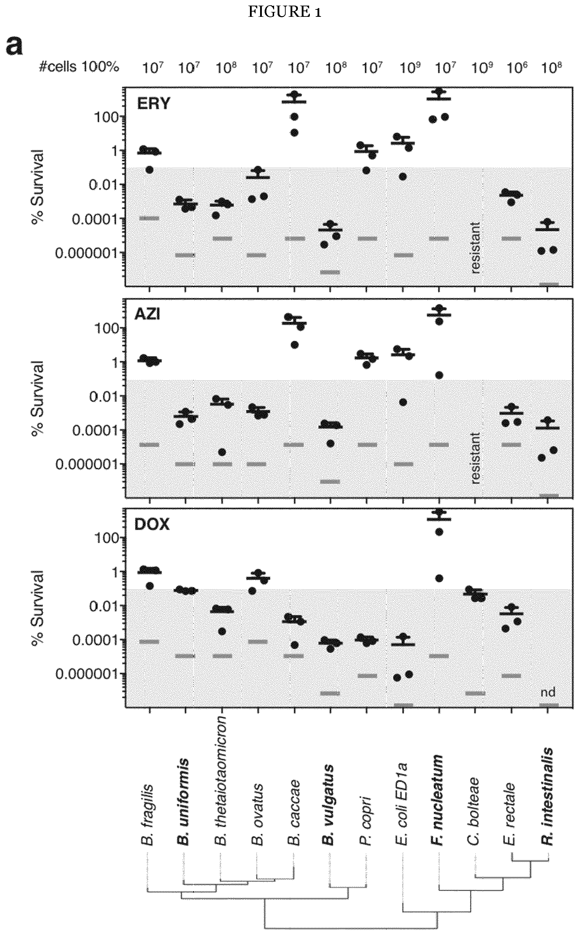

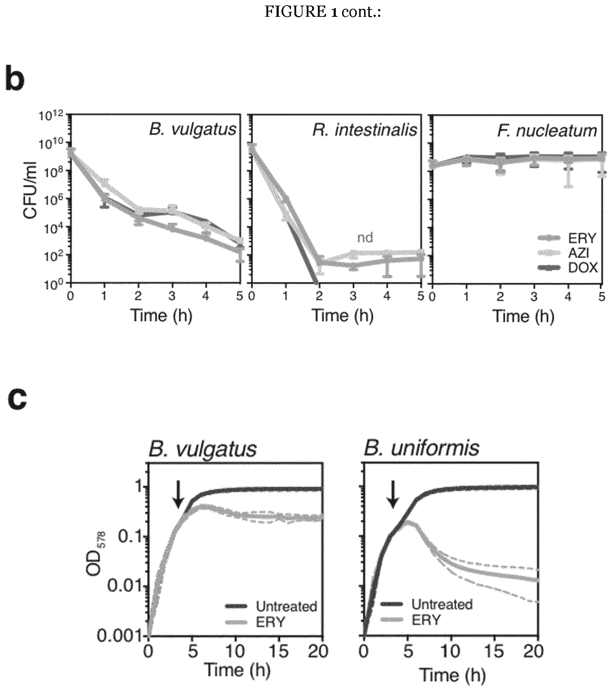

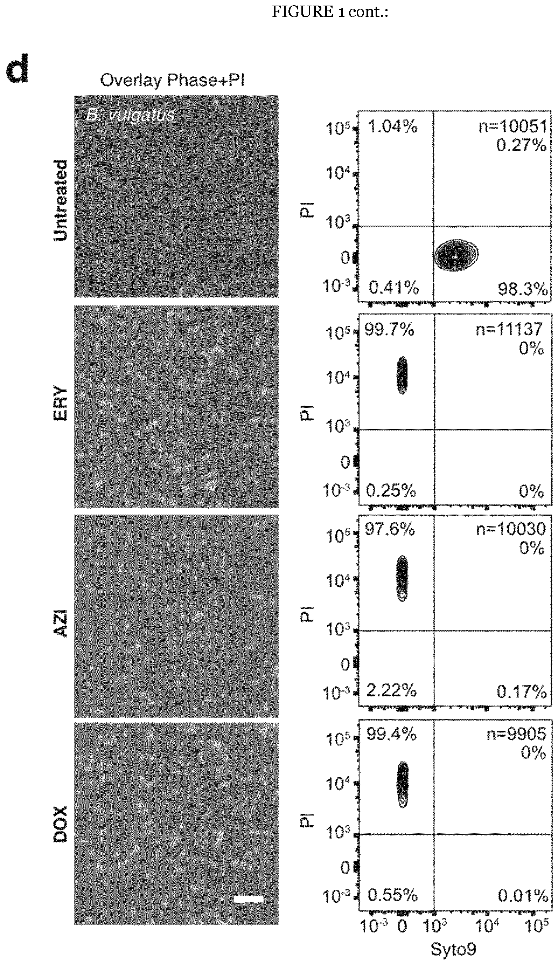

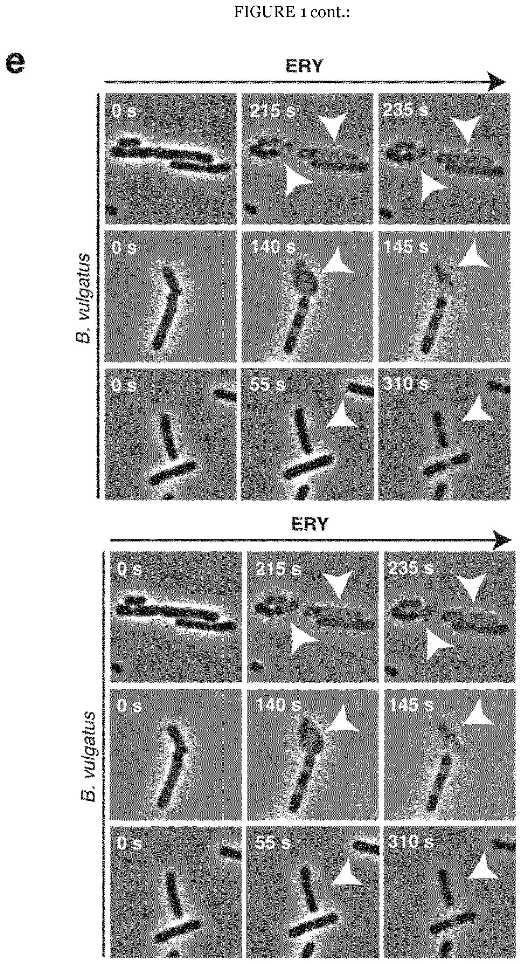

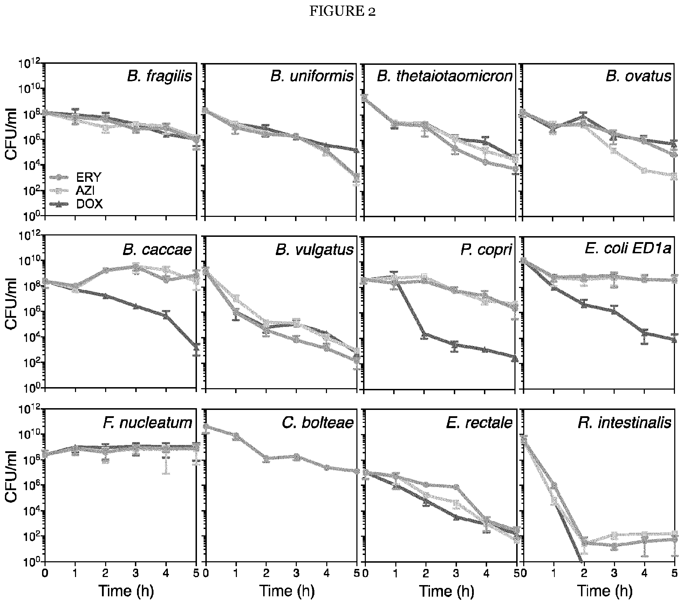

**Figure 1** **shows macrolides and tetracyclines kill human gut commensal species. a**. The survival of 12 abundant gut microbe species was measured after a 5-hour treatment with a 5-fold MIC of erythromycin (ERY), azithromycin (AZI) or doxycycline (DOX). The survival was assessed by counting CFUs/ml before and after antibiotic treatment. The number of CFUs/ml before treatment was set as 100%. The detection limit for each experiment (gray bar) and the bactericidal threshold (shaded area) are indicated. Species are plotted according to phylogeny (IQTree, Methods) and in bold are noted the species that are used in later panels. The graph shows the mean+SD of 3 independent experiments. **b.** Time-kill curves of *B. vulgatus, R. intestinalis* and *F. nucleatum* after antibiotic treatments. Survival was assessed by CFU counting over a 5 hour-treatment of ERY, AZI or DOX. This graph shows the mean±SD of 3 independent experiments. Nd: non-detectable. Time-kill curves for the other tested gut microbes can be found in Fig. 2. **c.** Erythromycin induces lysis of *B. vulgatus* and *B. uniformis. B. vulgatus* and *B. uniformis* were grown for 3 hours before addition (yellow) or not (black) of 15 µg/ml ERY treatment (5-fold MIC; yellow) as indicated by the arrow. Growth curves were acquired for 20 hours. This graph shows the mean DSD (dotted line) of 3 independent experiments. **d.** Live/dead staining of macrolide or tetracycline-treated *B*. *vulgatus.* The left panel shows an overlay of phase contrast and fluorescence microscopy images of propidium iodide (PI)-stained *B. vulgatus* before and 5 hrs after ERY, AZI or DOX treatment. Cultures were concentrated before imaging; the scale bar is 10 µm. The right panel shows the corresponding quantification of live/dead-stained cells by flow cytometry with Syto₉ on the x-axis (live cells) and PI on the y-axis (dead cells). Both the total number of measured events (n) and the percentage of cells found in each quadrant are indicated. **e.** Erythromycin induces blebbing, cytoplasmic shrinkage and lysis in *B. vulgatus* and *B. uniformis.* Phase contrast movies of *B. vulgatus* and *B. uniformis* were acquired after ERY treatment (5-fold MIC). Here shown are 3 frames of 3 images per strain (time indicated in the upper left corner; t=o when drug added). White arrows indicate blebs, cytoplasmic shrinkage and bacterial lysis; the scale bar is 5 µm.
**Figure 2** **shows time-kill curves of 12 abundant gut microbes after treatment with macrolides and tetracyclines.** Survival of 12 abundant gut microbes was assessed by CFU counting over a 5 hour-treatment of either ERY, AZI or DOX. This graph shows the mean±SD of 3 independent experiments.

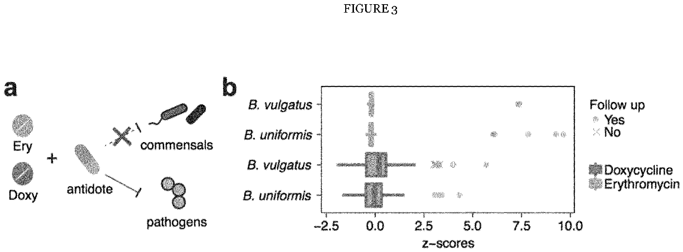

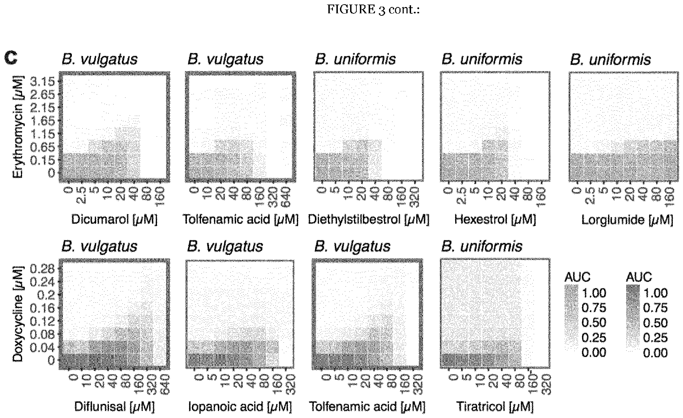

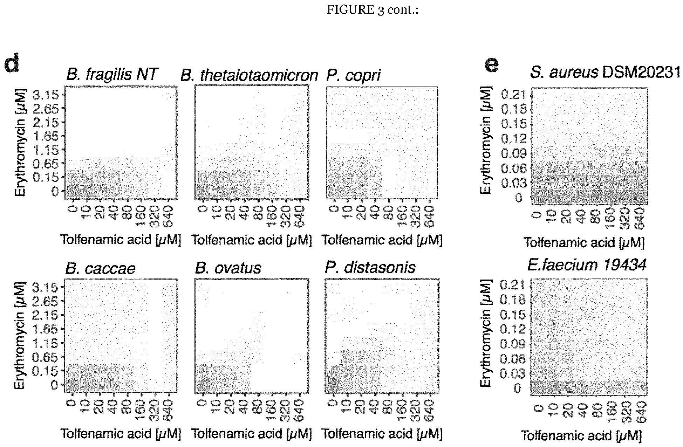

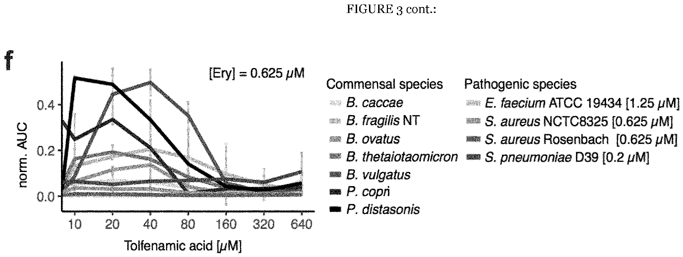

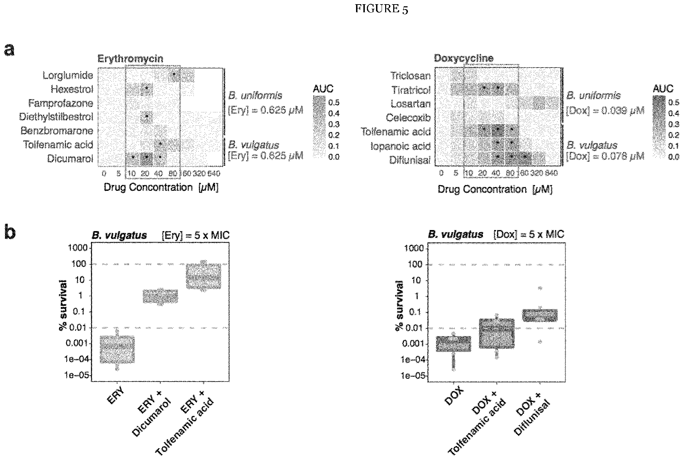

**Figure 3** **shows antidotes for selective protection of prevalent and abundant gut commensal species from macrolides and tetracyclines. a.** Schematic illustration of the screen concept: searching for antidote compounds that antagonize the antibacterial effect of erythromycin or doxycycline on commensal but not on pathogenic bacteria. **b.** Z-scores on bacterial growth (based on areas under the curve (AUCs)) for combinatorial drug exposure with antibiotic (ERY or DOX) and FDA-approved drug. Compounds that successfully rescued *B*. *vulgatus* and/or *B. uniformis* growth in the presence of the antibiotic (z-score > 3) are indicated in gray. The strongest hits (circles) were validated further in concentration-dependent assays (Fig. 5 a). For each antibiotic and each strain, ∼1200 drugs were tested in two replicates. Boxplots are plotted as in Fig. 1 d. **c.** For 9 of the validated antagonists, 8 x 8 checkerboard assays were performed to determine concentration ranges of the antagonistic interaction. Heat maps depict bacterial growth based on normalized median of AUCs of 4 replicates. Antagonistic interactions are framed in red, and pairs tested further in other commensal species in bold. **d.** Checkerboard assays confirm the ability of tolfenamic acid to protect further gut commensals from growth inhibition by erythromycin. Heat map as in **c,** but for **2** replicates. Antagonistic interactions are framed in red. **e.** Checkerboard of tolfenamic acid with erythromycin reveal neutral interactions in *S. aureus* and *E. faecium* (aerobic conditions). Heat maps as in c. **f.** Tolfenamic acid concentration-dependent rescue of commensal growth at clinical relevant erythromycin concentrations based on AUCs (anaerobic conditions). Erythromycin still retains its activity against pertinent pathogens such as *S. aureus* and *S. pneumoniae* (aerobic conditions). 0.625 µM correspond to ∼0.5 µg/ml erythromycin, which is in the range of the MIC breakpoints for *Staphylococcus* (1 µg/ml), *S. pneumoniae* (0.25 µg/ml) and *Streptococcus* groups A, B, C & G (0.25 µg/ml). Error bars depict standard deviation.

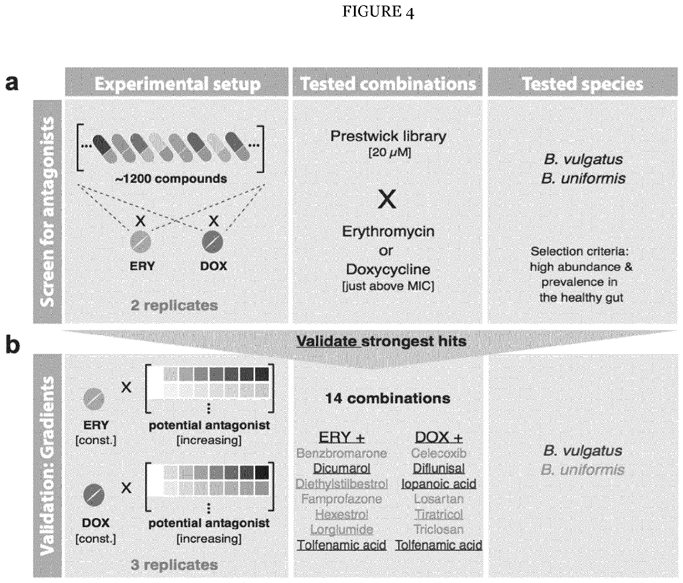

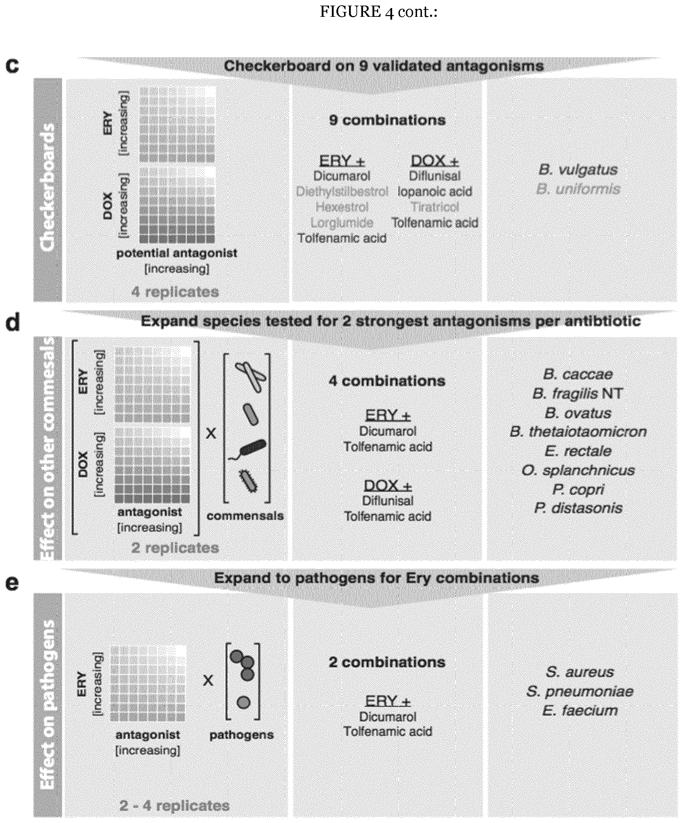

**Figure 4** **shows a schematic overview of the screen for microbiome-protective antibiotic antagonisms.** Workflow with decision process which antagonist to move on to next evaluation step.
**Figure 5** **shows validation of potential microbiome-protective antagonists. a.** Validation of the strongest antagonists in independent experiments. Erythromycin and doxycycline concentrations were kept constant ([ERY]=0.625 µM, [DOX] = 0.039 / 0.078 µM) and concentration ranges were tested for antagonist. Asterisks indicate that at least 25% of the bacterial growth (compared to no drug controls) could be rescued by the antagonist at a given concentration. Heat map depicts median AUCs across triplicates. **b.** Percentage of surviving *B*. *vulgatus* cells were determined after 5 h incubation with either erythromycin (3.25 µM) or doxycycline (0.4 µM) alone or in presence of the antagonist dicumarol (20 µM), tolfenamic acid (40 µM) or diflunisal (80 µM). Data is based on 3 replicates. Boxplots are plotted as in Fig. 1 d.

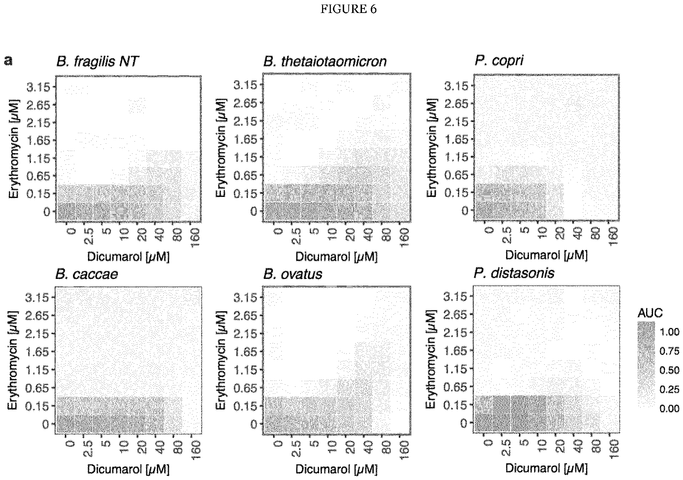

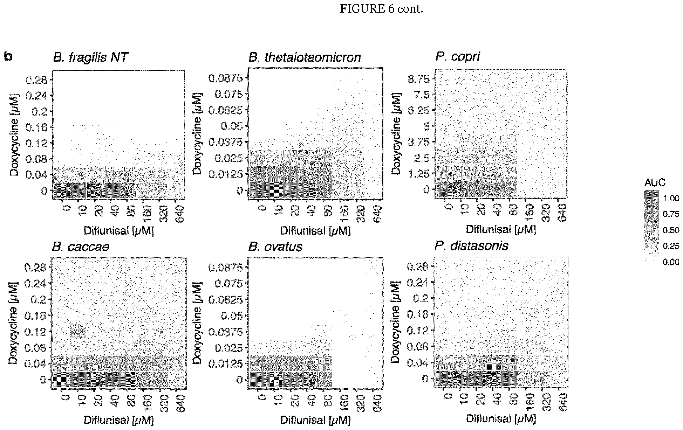

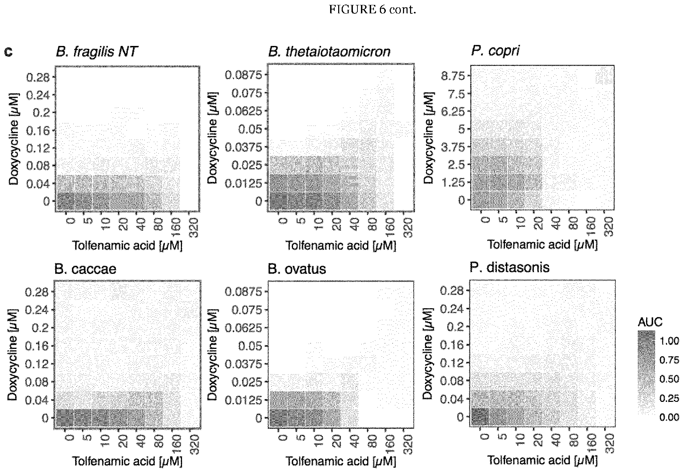

**Figure 6** **shows the effect of antidotes on further gut commensals.** 8 x 8 checkerboard assays to investigate if antidote is also protective for additional gut commensals for the following combinations: erythromycin and dicumarol (**a**), doxycycline and diflunisal (**b**) and doxycycline and tolfenamic acid (**c**). Heat map depicts bacterial growth based on median AUCs from two independent replicates. Red contours indicate antagonistic drug interactions.

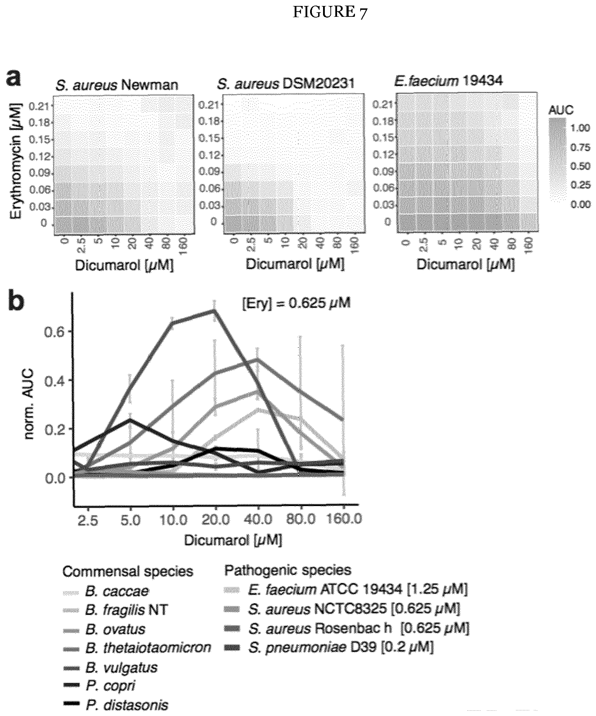

**Figure 7** **shows the effect of the antidote dicumarol on pathogens, relatively to commensal species. a.** Checkerboard assays for the drug combinations erythromycin-tolfenamic acid and erythromycin-dicumarol on the pathogens *S. aureus* (two different strains) and *E. faecium.* Heat map depict median normalized AUCs of checkerboard assays (at least three independent replicates). **b**. Dicumarol rescues commensal growth (based on median AUCs, N=2) at clinical relevant erythromycin concentrations in a concentration-dependent manner. Erythromycin still retains its activity against pertinent pathogens such as *S. aureus* and *S. pneumoniae* (based on median AUCs, N=3). Error bars depict standard deviation.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

**Growth conditions:** All experiments from this study were performed in an anaerobic chamber (Coy Laboratory Products Inc) (2% H₂, 12% CO₂, 86% N₂) and all materials and solutions used for these experiments were pre-reduced for at least 24 h before use unless specified otherwise. Bacteria used in this study were typically pre-cultured for 2 overnights: Cells were cultured in 5 ml modified Gifu Anaerobic Medium broth (MGAM) (HyServe GmbH & Co.KG, Germany, produced by Nissui Pharmaceuticals) and grown at 37°C overnight. The next day, cells were diluted 1/100 in 5 ml MGAM medium and grown at 37°C for a second overnight before starting the experiments.

**Quantitative assay for minimum inhibitory concentration determination with MICs test strips:** MICs test strips were purchased from Liofilchem or Oxoid. All MICs were measured under anaerobic growth conditions inside a Coy anaerobic chamber. Bacteria were precultured in MGAM for two overnights and cultures were diluted to OD₅₇₈ = 0.5. 50 µl of the diluted culture were spread on a MGAM agar plate and allowed to dry for 15 min. The MIC test strip was placed on the agar with sterile tweezers, allowing the part with the lowest concentration touch the agar first. Plates were incubated at 37°C inside the anaerobic chamber, at least overnight and longer depending on the species-specific growth requirements. After formation of a symmetrical inhibition ellipse, plates were taken out of the chamber and imaged under controlled lighting conditions (spImager S&P Robotics Inc.) using a 18 megapixel Canon Rebel T3i (Canon Inc. USA). MICs are directly determined from the strip scale at the point where the edge of the inhibition ellipse intersects the MIC test strip. All MICs were determined in duplicates. In cases of an eight-fold difference between the two values, a third replicate was done. In all cases, this resulted in a clear outlier (> 8-fold different from other two MICs) that was removed from the dataset.

**MIC comparison to ChEMBL and EUCAST databases:** Previously known MICs were extracted from the ChEMBL database (version 24) and EUCAST (obtained on May 14, 2018). Antibiotics from these two datasets were mapped to the inventor's dataset by name. Species were mapped using NCBI Taxonomy Identifiers and species names. For MICs from ChEMBL, a keyword-based approach was used to exclude experiments on species with mutations, deletions, insertions, etc. The EUCAST database contains a large number of reported MICs for each compound-species pair. The inventors collapsed these to a single value by calculating the median MIC.

Estimates on the abundance and prevalence of species in the healthy human gut microbiome were calculated using mOTUs v2 as follows: Relative species abundances were determined in 727 shotgun metagenomic samples from donors in the control groups of multiple studies from various countries and continents. Prior to taxonomic profiling, metagenomes were quality controlled using the MOCAT2-rtf procedure, which removed reads with ≥95% sequence identity and an alignment length of ≥45bp to the human genome hg₁₉. Taxonomic profiles were then created using mOTUs version 2.1.0 with parameters -l 75 ; -g 2; and -c. Afterwards relative abundances below 10⁻⁴ were set to zero and species with nonzero abundance in <5 samples discarded. For the retained 1,350 species, prevalence was defined as the percentage of samples with nonzero abundance; a prevalence cut-off of 1% was chosen to classify species into "rare" and "common" species. For all species in the MIC dataset, the inventors manually assessed their status as pathogenic or non-pathogenic species using encyclopaedic and literature knowledge. Pathogenic species that occur in more than 1% of healthy people (i.e. are designated as "common") were classified as "potentially pathogenic species" that can, for example, cause diseases in immunocompromised patients.

**Killing curves and survival assay:** Cells were precultured as described in the *growth conditions* section before being diluted to an OD₅₇₈=0.01 and grown for 2 h at 37°C (unless specified otherwise). Next, cells were diluted 1/2 in MGAM containing a 10-fold MIC of erythromycin, azithromycin or doxycycline (final antibiotic concentration is 5-fold MIC) and incubated in the presence of the antibiotic for 5 h at 37°C. At several time-points (0, 1h, 2h, 3h, 4h, 5h), 100 µl of cells were serial-diluted in PBS (10⁻¹ to 10⁻⁸ dilutions) and plated on MGAM-Agar plates for colony forming unit (CFU) counting. When no cells were detected using this method, a bigger volume of culture (up to 2 ml) was plated to be able to detect CFUs. Agar plates were incubated overnight at 37°C and colonies were counted the next day, either manually, for low CFU numbers, or using the *Analyze Particles* tool from ImageJ³⁵.

**Live/dead staining:** Cells were precultured as described in the *growth conditions* section before being diluted to an OD₅₇₈=0.01 and grown for 2 h at 37°C. Cells were next diluted 1/2 in MGAM containing 10-fold MIC of erythromycin, azithromycin or doxycycline (final concentration is 5-fold the MIC) and incubated in the presence of the antibiotic for 5 hours at 37°C. Then, cells were live/dead stained using the *LIVE*/*DEAD BacLight Bacterial viability and counting kit* (#L34856 Molecular Probes, ThermoFisher) according to the manufacturer's protocol before and after antibiotic treatment.

**Flow cytometry:** Stained cells were counted using a BD LSRFortessa™ flow cytometer. The forward and side scatter signals (488 nm) as well as the green and red fluorescent signals (488-530/30A filter and 561-610/20A filter, respectively) were acquired. The FSC/SSC detectors were set to logarithmic scale. The flow rate varied between 12 µl/min and 60 µl/min depending on the concentration of each sample, and the analysis was stopped when 10,000 target events were measured. Graphs were generated using the FlowJo V10.3 software (Treestar).

**Microscopy:** For live/dead imaging, stained cells were washed twice in 0.85% NaCl before being spotted on 0.85% NaCl + 1% agarose pads between a glass slide and a coverslip. For time-lapse imaging, cells were precultured as described in the *growth conditions* section. Cells were then diluted to an OD₅₇₈=0.01 and grown for 3 h at 37°C before being spotted on MGAM +1% agarose pads, supplemented or not with 15 µg/ml erythromycin (5-fold MIC) between a glass slide and a coverslip. Slides were sealed with valap and taken outside of the chamber for imaging. The imaging was performed using a Nikon Eclipse Ti inverted microscope, equipped with a Nikon DS-Qi2 camera, a Nikon Plan Apo Lambda 60X oil Ph₃ DM phase contrast objective and a Nikon HC mCherry filter set (Ex 562/40; DM 593; BA 641/75) to detect propidium iodide fluorescence. Images were acquired with the NIS-Elements AR4.50.00 software and processed with Fiji v.2.0.0-rc-68/1.52h.

**Growth curves:** Cells were precultured as described in the *growth conditions* section. Then, cells were diluted to an OD₅₇₈=0.01 in a 96-well plate sealed with a breathable membrane (Breathe-Easy®) and grown for 2 h. Next, erythromycin was added to the culture to a final concentration of 15 µg/ml (5-fold MIC) and growth curves were acquired for 20 h using a microplate spectrophotometer (EON, Biotek) by measuring the OD₅₇₈ every hour after 30 sec of linear shaking.

The examples show:

### Example 1: Antibacterial effects on gut microbiota

The standard way to determine whether an antibiotic has bactericidal or bacteriostatic activity is to calculate time-kill curves, where the bacterial survivors are counted on agar at various time-points after drug treatment. If, over a significant period of antibiotic treatment (ranging from a 5 to 24 hours), the number of colony forming units (CFU)/ml of culture decreases by more than 99.9%, the antibiotic is considered bactericidal.

The inventors assessed the survival of 12 abundant gut microbes over a 5-hour treatment of either a macrolide (erythromycin or azithromycin) or a tetracycline (doxycycline) at 5 x MIC (Fig 1a + b, Fig. 2).

Surprisingly, about half of the tested species decreased in survival by >99.9%, confirming that these drugs are bactericidal to several abundant gut microbes. To confirm this further, the inventors tested the viability of *B. vulgatus* and *E. coli* EDia upon erythromycin, azithromycin or doxycycline treatments using live/dead staining. Microscopy and flow cytometry assessment of live/dead bacteria corroborated the initial observations (Fig. 1 c). As tetracylines are considered bona-fide bacteriostatic drugs in *E. coli,* the inventors were surprised to see that doxycycline effectively killed the commensal *E. coli* EDia (Fig. 1 a).

The inventors also noticed that *B. vulgatus* and *B. uniformis* cultures decreased density in the presence of erythromycin (Fig. 1 d). The inventors confirmed by time-lapse microscopy that this was due to lysis. Erythromycin caused cell shape defects, including blebbing, cytoplasmic shrinkage, and ultimately cell lysis in both *B. vulgatus* and *B. uniformis* (Fig. 1 e). Altogether, the differential bactericidal activity of macrolides and tetracyclines on gut commensals could provide an explanation for the strong effects these drug classes have on the gut microbiota composition of human individuals.

### Example 2: Screen for microbiome-protective antibiotic antagonism

The inventors reasoned that it should be possible to identify drugs that selectively antagonize the effect of antibiotics on gut microbes, while retaining activity against pathogens. Therefore, the inventors screened the Prestwick library to identify antagonizing compounds to erythromycin or doxycycline on two abundant and prevalent gut microbes, *B. vulgatus* and *B. uniformis.*

*Preparation of screening plates.* The Prestwick Chemical Library was purchased from Prestwick Chemical Inc. and drugs were re-arrayed, diluted and stored in 96 well format as described before. The inventors prepared drug plates (2 x drug concentration) in MGAM medium and stored them at -30°C. For each experiment, drug plates were thawed, supplemented with the respective antibiotic solution (freshly prepared in MGAM) and pre-reduced in the anaerobic chamber overnight. All rearranging and aliquoting steps were done using the Biomek FXP (Beckman Coulter) system.

*Inoculation and screening conditions.* Strains were grown twice overnight, the second overnight culture was diluted in MGAM to reach OD_{578 nm} 0.04 (4 x the desired starting OD). 25 µl of the diluted cultures were used to inoculate wells containing 50 µl of 2x concentrated Prestwick drug and 25 µl of the 4x concentrated antibiotic using the semi-automated, 96-well multi-channel pipette ep*Motion*96 (Eppendorf). Each well contained 1% DMSO, 20 µM of the Prestwick drug and a species-specific antibiotic concentration that was just inhibitory for the respective strain (0.625 µM for erythromycin, 0.04 µM doxycycline for *B. uniformis* and 0.08 µM doxycycline for *B. vulgatus*). Plates were sealed with breathable membranes (Breathe-Easy®) and OD₅₇₈ was measured hourly after 30 sec of linear shaking with a microplate spectrophotometer (EON, Biotek) and an automated microplate stacker (Biostack 4, Biotek) fitted inside a custom-made incubator (EMBL Mechanical Workshop). Growth curves were collected up to 24 h. For each antibiotic, each species was screen in biological duplicates. All experiments included control wells of unperturbed growth (32 wells per run) and control wells for growth in the presence of the antibiotic only (8 wells per plate).

*Analysis pipeline and hit calling:* All growth curves within a plate were truncated at the transition time from exponential to stationary phase and converted to normalized AUCs using in-run control wells (no drug) as described before. The inventors then calculated z-scores based on these normalized AUCs, removed replicates with 8-fold differences in z-scores to eliminate noise effects, computed mean z scores across the two replicates and selected combinations with mean z-scores > 3. This selection included 19 potential antibiotic antagonists and the inventors followed up on 14 of them (7 potential erythromycin and 7 potential doxycycline antagonists in either *B**.** vulgatus* or *B. uniformis* - see Fig. 4) in independent experiments.

*Validation of microbiome-protective antagonists:* In a first step, the inventors kept the erythromycin/doxycycline concentration constant (0.625 µM for erythromycin, 0.078 µM (*B*. *vulgatus)*/ 0.039 µM (*B. uniformis*) for doxycycline) and tested concentration gradients of the potential antagonists with ranges depending on the antagonist's solubility. Compounds were purchased from independent vendors and dissolved at 100x starting concentration in DMSO. Eight 2-fold serial dilutions were prepared in 96-well plates with each row containing a different antagonist, sufficient control DMSO wells and wells with just the respective antibiotic ('antibiotic-only' control). These master plates were diluted in MGAM medium (50 µl) to 2 x assay concentration and 25 µl freshly prepared antibiotic solution (4x test concentration) was added. Plates were pre-reduced overnight in an anaerobic chamber and inoculated with 25 µl of overnight cultures (prepared as described under "Growth conditions") to reach a starting OD₅₇₈ of 0.01 and 1% DMSO concentration. Growth was monitored hourly for 24 h after 30 sec of linear shaking. Experiments were performed in biological triplicates. For analysis, growth curves were converted into normalized AUCs (see above). The inventors accounted for residual growth in the presence of the antibiotic by subtracting the median normalized AUCs of the 'antibiotic-only' control per plate. The inventors computed medians across triplicates and considered a normalized AUC > 0.25 as concentration-dependent growth rescue by the antagonist.

*Checkerboard assays for anaerobic commensals:* Validated antagonists were further investigated in 8x8 checkerboard assays, where both antibiotics and antagonists were titrated against each other. Such assays were first performed for the commensals that were originally screened (i.e. *B. vulgatus* and *B. uniformis* - 4 replicates) and later expanded towards 6 further gut microbes (*B. caccae, B. fragilis* NT, *B. ovatus, B. thetaiotaomicron, P. copri, P. distasonis* - 2 replicates). For vertical gradients, 2-fold serial dilutions of the antagonists were prepared first in 100x in DMSO and diluted in MGAM as described above (section 'Validation of microbiome-protective antagonists'). Horizontal antibiotic dilution series were freshly prepared in MGAM at 4x final concentration in equidistant concentration steps. Both, vertical and horizontal dilution series were combined (50 µl of the antagonist gradients (2x) and 25 µl of the antibiotic gradients (4x)) in 96 well plates. Plates were pre-reduced under anaerobic conditions overnight, inoculated with 25 µl of diluted overnight culture (at 4x starting OD) and sealed with breathable membrane (Breathe-Easy®). Bacterial growth was monitored once per hour for 24 hours after 30 sec linear shaking (Eon + Biostack 4, Biotek) under anaerobic conditions.

Growth curves were converted into normalized AUCs as described using in-plate controls to define unperturbed growth.

*Checkerboard assays for pathogens under aerobic conditions:* For three pathogens (*S. aureus* Newman ATCC 13420, *S. aureus* subsp. aureus Rosenbach 1884 DSM20231 and *E*. *faecium* ATCC19434) 8x8 checkerboard assays were performed in transparent 384 well plates (Greiner BioOne GmbH), with each well containing 30 µl (50 µl for *S. pneumoniae*) in total. *S*. *aureus* strains were grown in tryptic soy broth, *E. faecium* in BHI medium. Antagonistic drug concentrations were 2-fold distant. Cell were inoculated at initial OD₅₉₅ₙₘ ∼0.01 from an overnight culture. Plates were sealed with breathable membranes (Breathe-Easy), incubated at 37°C (Cytomat 2, Thermo Scientific) with continuous shaking and OD₅₉₅ₙₘ was measured every 30 minutes for 16 hours in a Filtermax F5 multimode plate reader (Molecular Devices). Growth was evaluated at the transition to stationary phase (9 hours for *S. aureus,* 12 hours for *E. faecium*). All experiments were done at least in 2 biological replicates and 2 technical replicates. For *S. pneumoniae* D₃₉, the inventors only tested concentration gradients of the potential antagonists in constant concentration of the antibiotics in BHI medium.

**Table 1: Screen for microbiome-protective antagonists to erythromycin/tetracycline**

| **Drug** | **Antibiotic** | **Strain** | **Mean z-score** | **Validation** |
|---|---|---|---|---|
| Hexestrol | Erythromycin | B.uniformis | 9.630 | TRUE |
| Diethylstilbestrol | Erythromycin | B.uniformis | 9.258 | TRUE |
| Famprofazone | Erythromycin | B.uniformis | 7.832 | FALSE |
| Tolfenamic acid | Erythromycin | B.vulgatus | 7.381 | TRUE |
| Dicumarol | Erythromycin | B.vulgatus | 7.313 | TRUE |
| Lorglumide sodium salt | Erythromycin | B.uniformis | 6.106 | TRUE |
| Benzbromarone | Erythromycin | B.uniformis | 6.034 | FALSE |
| Iopanoic acid | Doxycycline | B.vulgatus | 5.698 | TRUE |
| Tiratricol, 3,3',5-triiodothyroacetic acid | Doxycycline | B.uniformis | 4.333 | TRUE |
| Tolfenamic acid | Doxycycline | B.vulgatus | 4.000 | TRUE |
| Losartan | Doxycycline | B.uniformis | 3.421 | FALSE |
| Diethylstilbestrol | Doxycycline | B.vulgatus | 3.296 | NA |
| Celecoxib | Doxycycline | B.uniformis | 3.275 | FALSE |
| Diflunisal | Doxycycline | B.vulgatus | 3.265 | TRUE |
| Efavirenz | Doxycycline | B.vulgatus | 3.138 | NA |
| Diclazuril | Doxycycline | B.vulgatus | 3.117 | NA |
| Triclosan | Doxycycline | B.uniformis | 3.066 | FALSE |
| Clonixin Lysinate | Doxycycline | B.vulgatus | 3.061 | NA |
| Meclofenamic acid sodium salt monohydrate | Doxycycline | B.vulgatus | 3.010 | NA |

| | | | | |
|---|---|---|---|---|
| Abbreviation: NA = not available | | | | |

### Example 3: Validation of microbiome-protective antibiotic antagonisms

Of the 19 identified hits (Fig. 3b, Table 1), the inventors tested the 14 candidates with the strongest activity in a concentration-dependent manner (Fig. 5 a). Nine retained antagonistic effects over a broader concentration range, which the inventors confirmed by checkerboard assays (Fig. 3 c). The antidotes that showed the strongest antagonisms were the anticoagulant drug dicumarol, and two non-steroidal anti-inflammatory drugs, tolfenamic acid and diflusinal. While dicumarol rescued *B. vulgatus* from erythromycin and diflusinal from doxycycline, tolfenamic acid was able to protect *B. vulgatus* from both drugs.

In addition, these interactions were able to at least partially rescue the killing of *B*. *vulgatus* by erythromycin and doxycycline (Fig. 5 b). The inventors then probed these 3 drugs for their ability to protect other abundant gut commensals and confirmed that both dicumarol and tolfenamic acid were able to counteract erythromycin on several species (Fig. 3d, Fig. 6). In contrast, both drugs did not affect the potency of erythromycin on *Staphylococcus aureus* and *Streptococcus pneumoniae,* pathogens against which erythromycin is routinely prescribed (Fig. 3e, Fig. 7 a). For example, tolfenamic acid and dicumarol at concentration ranges of 5-40 µM could rescue the growth of 5/7 tested abundant gut commensal species at clinically relevant erythromycin concentrations (Fig. 3f, Fig. 7 b). Altogether, the inventor's data provides antidotes that specifically mask the collateral damage of antibiotics on commensals.

## Claims

1. A compound, or a pharmaceutically acceptable salt thereof, for use in treating and/or preventing dysbiosis, and/or for use in antagonizing an antibacterial effect of at least one second compound in at least one first bacterium, wherein said compound, or the pharmaceutically acceptable salt thereof, is capable of antagonizing an antibacterial effect of at least one second compound in at least one first bacterium, and wherein said compound, or the pharmaceutically acceptable salt thereof, is not capable of antagonizing an antibacterial effect of said at least one second compound in at least one second bacterium.

2. The compound, or the pharmaceutically acceptable salt thereof, according to claim 1, wherein said compound, or the pharmaceutically acceptable salt thereof, is selected from an anticoagulant drug, such as Dicumarol, a non-steroidal anti-inflammatory drug, such as Famprofazone, Tolfenamic acid, Celecoxib, Clonixin, or Meclofenamic acid, an Uricosuric, such as Benzbromarone, a nonsteroidal estrogen compound, such as Diethylstilbestrol or Hexestrol, an antagonist of the Cholecystokinin CCKA receptor, such as Lorglumide, a salicylic acid derivative, such as Diflunisal, a cholecystographic agent, such as Iopanoic acid, an angiotensin II receptor blocker, such as Losartan, a thyroid hormone analogue, such as Tiratricol, an antibacterial, antifungal and/or antibacterial agent, such as Triclosan or Diclazuril, a reverse transcriptase inhibitor, such as Efavirenz, a Ca-channel inhibitor, a human-targeted drug, and a food additive, or a pharmaceutically acceptable salt thereof.

3. A pharmaceutical composition, comprising:
(i) at least one first compound, or a pharmaceutically acceptable salt thereof, wherein said at least one first compound is capable of antagonizing an antibacterial effect of at least one second compound in a first bacterium, and wherein said at least one first compound is not capable of antagonizing an antibacterial effect of said at least one second compound in a second bacterium;
(ii) at least one second compound, or a pharmaceutically acceptable salt thereof, and
(iii) optionally, a pharmaceutically acceptable additive, carrier, diluent, solvent, filter, lubricant, excipient, binder, and/or stabilizer.

4. The pharmaceutical composition according to claim 3, wherein said at least one first compound (i) is selected from an anticoagulant drug, such as Dicumarol, a non-steroidal anti-inflammatory drug, such as Famprofazone, Tolfenamic acid, Celecoxib, Clonixin, or Meclofenamic acid, an Uricosuric, such as Benzbromarone, a nonsteroidal estrogen compound, such as Diethylstilbestrol or Hexestrol, an antagonist of the Cholecystokinin CCKA receptor, such as Lorglumide, a salicylic acid derivative, such as Diflunisal, a cholecystographic agent, such as Iopanoic acid, an angiotensin II receptor blocker, such as Losartan, a thyroid hormone analogue, such as Tiratricol, an antibacterial, antifungal and/or antibacterial agent, such as Triclosan or Diclazuril, a reverse transcriptase inhibitor, such as Efavirenz, a Ca-channel inhibitor, a human-targeted drug, and a food additive, or a pharmaceutically acceptable salt thereof.

5. The compound according to claim 1 or 2, or the pharmaceutical composition according to claim 3 or 4, wherein said at least one second compound is an antibiotic, preferably wherein said antibiotic is selected from a macrolide, a penicillin, a cephalosporin, a quinolone, a fluoroquinolone, a sulphonamide, a tetracycline, a monobactam, a carbapnem, an aminoglycoside, a rifamycin, a beta-lactam, an ansamycin, an oxazolidinone, a strepotgramin, a glycopeptide, a polypeptide, and an arsphenamine, or a pharmaceutically acceptable salt thereof, more preferably wherein said antibiotic is selected from erythromycin, azithromycin, clarithromycin, dirithromycin, clindamycin, doxycycline, minocycline, tigecyline, trimethoprim, pyocyanin, vancomycin, streptomycin, dihydrostreptomycin, amikacin, apramycin, arbekacin, astromicin, bekanamycin, dibekacin, framycetin, gentamicin, hygromycin, isepamicin, kanamycin, neomycin, netilmicin, paromomycin, rhodostreptomycin, ribostamycin, sisomycin, spectinomycin, tobramycin, verdamicin, polymixin, glycylcycline, carbapenem, carbacephem, chloramphenicol, clindamycin, lincomycin, daptomycin, novobiocin, clindamycin, ethambutol, fosfomycine, fusidic acid, furazolidone, isoniazid, linezolide, metronidazole, mupirocin, nitrofurantoin, platensimycine, pyrazinamide, quinupristine, benzalkonium, dalfopristine, rifampine, rifampicin, rifabutin, rifaximin, tinidazole, viomycin, and capreomycin, or a pharmaceutically acceptable salt thereof.

6. The pharmaceutical composition according to any one of claims 3 to 5, wherein said at least one first compound (i) is Dicumarol, Tolfenamic acid, Benzbromarone, Diethylstilbestrol, Famprofazone, Hexestrol, or Lorglumide, or a pharmaceutically acceptable salt thereof, and said at least one second compound is an antibiotic, preferably wherein said at least one second compound (ii) is a macrolide, such as erythromycin, azithromycin, clarithromycin, dirithromycin, or clindamycin, or a pharmaceutically acceptable salt thereof.

7. The pharmaceutical composition according to any one of claims 3 to 5, wherein said at least one first compound (i) is Diflunisal, Tolfenamic acid, Iopanoic acid, Tiratricol, Celecoxib, Losartan, Triclosan, Clonixin, Diethylstilbestrol, Meclofenamic acid, Diclazuril, Efavirenz, or a pharmaceutically acceptable salt thereof, and said at least one second compound (ii) is an antibiotic, preferably wherein said at least one second compound is a tetracycline, such as doxycycline, minocycline, tigecyline, or lymecyclin, or a pharmaceutically acceptable salt thereof.

8. The compound according to claim 1 or 2, or the pharmaceutical composition according to any one of claims 3 to 7, wherein said at least one first bacterium is a Gram-positive bacterium or a Gram-negative bacterium, preferably wherein said at least one first bacterium is a member of a genus selected from *Bacteroides, Eubacterium, Faecalibacterium, Odoribacter, Prevotella, Parabacteroides, Ruminococcus, Roseburia, Bifidobacterium, Coprococcus, Dorea, Blautia, Clostridium, Escherichia, Streptococcus, Collinsella, Bilophila, Akkermansia, Veillonella, Haemophilus, Desulfovibrio, Fusobacterium, Lactobacillus, Alistipes,* and *Butyrivibrio.*

9. The compound according to claim 1 or 2, or the pharmaceutical composition according to any one of claims 3 to 8, wherein said at least one second bacterium is a Gram-positive bacterium or a Gram-negative bacterium, and wherein said at least one second bacterium is a member of a genus selected from *Enterococcus, Staphylococcus, Enterobacter, Streptococcus, Pseudomonas, Escherichia, Salmonella, Helicobacter, Neisseria, Campylobacter, Chlamydia, Clostridia, Citrobacter, Vibrio, Treponema, Mycobacterium, Klebsiella, Actinomyces, Bacteroides, Bordetella, Borrelia, Brucella, Corynebacteria, Diplococcus, Fusobacterium, Leptospira, Listeria, Pasteurella, Proteus, Rickettsia, Shigella, Yersinia, Parabacteroides, Odoribacter, Faecalibacteria, Collinsella, Eggerthella, Lactonifactor, Pediococcus, Leuconostoc, Lactococcus, Roseburia, Coliform, Bacillus, Franscicella, Acinetobacter, Legionella, Actinobacillus, Coxiella, Kingella kingae, Haemophilus,* and combinations thereof, optionally wherein said at least one second bacterium is an antibiotic-resistant bacterium and/or a multi-drug resistant bacterium.

10. The compound according to claim 1 or 2, or the pharmaceutical composition according to any one of claims 3 to 9, wherein said at least one second compound is for use in the prevention and/or treatment of a bacterial infection, preferably wherein said bacterial infection is selected from an infection of the gastrointestinal tract, an infection of the urogenital tract, an infection of the upper and lower respiratory tract, rhinitis, tonsillitis, pharyngitis, bronchitis, pneumonia, an infection of the inner organs, nephritis, hepatitis, peritonitis, endocarditis, meningitis, osteomyelitis, an infection of the eyes, an infection of the ears, a cutaneous infection, a subcutaneous infection, an infection after burn, diarrhea, colitis, pseudomembranous colitis, a skin disorder, toxic shock syndrome, bacteremia, sepsis, pelvic inflammatory disease, an infection of the central nervous system, wound infection, intra-abdominal infection, intravascular infection, bone infection, joint infection, acute bacterial otitis media, pyelonephritis, deep-seated abscess, and tuberculosis.

11. The compound according to claim 1 or 2, or the pharmaceutical composition according to any one of claims 3 to 10, wherein said compound or said pharmaceutical composition is in liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, or room spray, optionally wherein said components (i) and (ii) of said pharmaceutical composition are administered to a subject simultaneously, separately, or sequentially.

12. The compound according to claim 1 or 2, or the pharmaceutical composition according to any one of claims 3 to 11, wherein said subject is a mammal, such as a mouse, rat, guinea pig, rabbit, cat, dog, monkey, or preferably a human, such as a human patient, optionally wherein said compound or said pharmaceutical composition is administered to said subject in a therapeutically effective amount, thereby preventing and/or treating a disease in said subject, and/or preventing a modification of the microbiome of said subject.

13. A compound according to claim 1 or 2, or a pharmaceutical composition according to any one of claims 3 to 12, for use in the prevention and/or treatment of a disease in a subject, and/or for use in the prevention of a modification of the microbiome of said subject, optionally wherein said disease is dysbiosis.

14. A compound according to claim 1 or 2 or 13, or a pharmaceutical composition according to any one of claims 3 to 13, wherein said compound or said pharmaceutical composition is administered to a subject, thereby modifying the growth of bacterial cells in said subject, wherein said modifying results in the prevention and/or treatment of a disease in said subject, and/or in the prevention of a modification of the microbiome of said subject.

15. A method for preventing an adverse effect on a gut microbiome using a compound according to claim 1 or 2, 13 or 14, or a pharmaceutical composition according to any one of claims 3 to 14, the method comprising administering to a subject an effective amount of a compound according to claim 1 or 2, 13 or 14, or a pharmaceutical composition according to any one of claims 3 to 14, thereby treating the disease in the subject, optionally wherein said method prevents of a modification of the microbiome of said subject, preferably wherein said disease is dybiosis.
